(19) Europäisches Patentamt / European Patent Office / Office européen des brevets

(11) **EP 3 238 632 B1**

(12) **EUROPEAN PATENT SPECIFICATION**

(45) Date of publication and mention
of the grant of the patent:
**07.08.2019 Bulletin 2019/32**

(21) Application number: **15872418.7**

(22) Date of filing: **07.10.2015**

(51) Int Cl.:
*A61B 8/12* (2006.01)          *A61B 8/14* (2006.01)
*A61B 8/00* (2006.01)          *G01S 7/52* (2006.01)
*A61B 8/08* (2006.01)

(86) International application number:
**PCT/JP2015/078548**

(87) International publication number:
**WO 2016/103849 (30.06.2016 Gazette 2016/26)**

(54) **ULTRASOUND OBSERVATION APPARATUS, METHOD FOR OPERATING ULTRASOUND OBSERVATION APPARATUS, AND PROGRAM FOR OPERATING ULTRASOUND OBSERVATION APPARATUS**

ULTRASCHALLBEOBACHTUNGSVORRICHTUNG, VERFAHREN ZUM BETRIEB DER ULTRASCHALLBEOBACHTUNGSVORRICHTUNG UND PROGRAMM ZUM BETRIEB DER ULTRASCHALLBEOBACHTUNGSVORRICHTUNG

APPAREIL D'OBSERVATION À ULTRASONS, PROCÉDÉ DE FONCTIONNEMENT POUR APPAREIL D'OBSERVATION À ULTRASONS, ET PROGRAMME DE FONCTIONNEMENT POUR APPAREIL D'OBSERVATION À ULTRASONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.12.2014 JP 2014259474**

(43) Date of publication of application:
**01.11.2017 Bulletin 2017/44**

(73) Proprietor: **Olympus Corporation**
**Tokyo 192-8507 (JP)**

(72) Inventor: **MIYAKI, Hironaka**
**Tokyo 192-8507 (JP)**

(74) Representative: **Gunzelmann, Rainer**
**Wuesthoff & Wuesthoff**
**Patentanwälte PartG mbB**
**Schweigerstraße 2**
**81541 München (DE)**

(56) References cited:
**WO-A1-2007/003058          WO-A1-2014/054469**
**WO-A1-2014/192954          JP-A- 2010 246 640**
**JP-A- 2010 246 640          US-A1- 2013 012 818**
**US-A1- 2013 035 594**

**Description**

Field

The present invention relates to an ultrasound observation apparatus for observing tissue of an observation target using ultrasound, a method for operating the ultrasound observation apparatus, and a program for operating the ultrasound observation apparatus.

Background

A conventional technology, which makes a correction for compensating a reception signal for the frequency-dependent attenuation of living tissue in an ultrasound observation apparatus that observes tissue of an observation target using ultrasound, has been known (refer to, for example, Patent Literature 1). In this technology, an ultrasound image is formed using a reception signal obtained by sequentially performing a dynamic correction process in accordance with the depth of a receiving point and a pulse compression process on a reflected wave from a subject.

US 2013/035594 A1 concerns an ultrasonic observation apparatus including a reference spectrum storage unit that stores a first reference spectrum in a first reception depth range and a second reference spectrum in a second reception depth range obtained based on a frequency of an ultrasonic wave received from a reference reflector, a frequency analyzer that calculates a frequency spectrum by analyzing a frequency of the received ultrasonic wave, and a corrected frequency spectrum calculator that calculates a corrected frequency spectrum by determining whether a reception depth of the frequency spectrum calculated by the frequency analyzer is the first reception depth range or the second reception depth range, and obtaining a difference, in a case of the first reception depth range, between the first reference spectrum and the frequency spectrum and a difference, in a case of the second reception depth range, between the second reference spectrum and the frequency spectrum.

Citation List

Patent Literature

Patent Literature 1: JP 2010-246640 A

Summary

Technical Problem

However, in the technology described in the above-mentioned Patent Literature 1 does not consider changes in attenuation factor up to a region of interest that is away from the surface of a probe that transmits and receives ultrasound, when a correction is made to a reception signal of the region of interest. Hence, its application is possible only when the attenuation factors of an observation target are uniform, and it is difficult to accurately identify the tissue characteristics of an observation target with nonuniform attenuation factors.

The present invention has been made in view of the foregoing, and an object of the invention is to provide an ultrasound observation apparatus that enables accurate identification of tissue characteristics of an observation target with nonuniform attenuation factors, a method for operating the ultrasound observation apparatus, and a program for operating the ultrasound observation apparatus.

Solution to Problem

In order to solve the above described problems and achieve the object, an ultrasound observation apparatus according to claim 1 is provided.

In the ultrasound observation apparatus according to the above-described invention, the feature correction unit is configured to calculate a sum of the optimum attenuation factor of each of the divided regions present between the surface of the ultrasound transducer and the sampling point, the optimum attenuation factor being multiplied by a weight indicating a round-trip distance in a depth direction in each of the divided regions, to calculate the cumulative attenuation factor at the sampling point.

In the ultrasound observation apparatus according to the above-described invention, in two of the divided regions which are adjacent along a depth direction, one of the two divided regions located more distant from the ultrasound transducer has a length in the depth direction equal to or more than a length in the depth direction of the other of the two divided regions located closer to the

2

ultrasound transducer.

In the ultrasound observation apparatus according to the above-described invention, the attenuation factor setting unit is configured to calculate a statistical dispersion of the preliminarily corrected feature for each of the attenuation factor candidate values, and set an attenuation factor candidate value having a minimal statistical dispersion as the optimum attenuation factor.

The ultrasound observation apparatus according to the above-described invention further includes a feature image data generation unit configured to generate feature image data for showing information on the corrected feature together with the ultrasound image.

In the ultrasound observation apparatus according to the above-described invention, the feature calculation unit is configured to approximate each of the frequency spectra by an n-th order polynomial (n is a positive integer) to calculate the features.

In the ultrasound observation apparatus according to the above-described invention, the feature calculation unit is configured to approximate a predetermined frequency band in the frequency spectrum by a linear expression to calculate the features that are one or more of an intercept of the linear expression, a slope of the linear expression, and a midband fit as a value of the linear expression at an intermediate frequency in the frequency band, and that include one of the slope and the midband fit. The attenuation factor setting unit is configured to set the optimum attenuation factor based on the one of the slope and the midband fit.

In the ultrasound observation apparatus according to the above-described invention, the attenuation factor setting unit is configured to set the optimum attenuation factor based on the slope when the slope is the features, and set the optimum attenuation factor based on the midband fit when the midband fit is the features.

A method for operating an ultrasound observation apparatus according to claim 9 is provided. The ultrasound observation apparatus is configured to generate an ultrasound image based on an ultrasound signal acquired by an ultrasound probe, the ultrasound probe having an ultrasound transducer configured to transmit ultrasound to an observation target and receive the ultrasound reflected from the observation target. The method includes: a frequency analysis step of, by a frequency analysis unit, analyzing a frequency of the ultrasound signal to calculate a plurality of frequency spectra in accordance with reception depths and receiving directions of the ultrasound signal; a feature calculation step of, by a feature calculation unit, calculating features of the plurality of frequency spectra; an attenuation factor setting step of, by an attenuation factor setting unit, using each of a plurality of attenuation factor candidate values per unit length and per unit frequency that give different attenuation characteristics when the ultrasound propagates through the observation target, in each of divided regions obtained by dividing the ultrasound image into a plurality of regions, to perform attenuation correction on the features of the frequency spectra for removing an influence of the ultrasound, and thereby calculating a preliminarily corrected feature of each of the frequency spectra for each of the attenuation factor candidate values, and setting an optimum attenuation factor for the observation target among the plurality of attenuation factor candidate values based on a calculation result; and a feature correction step of, by a feature correction unit, calculating a cumulative attenuation factor per unit frequency at a sampling point by using an optimum attenuation factor of a divided region present between a surface of the ultrasound transducer and the sampling point among optimum attenuation factors set respectively for the divided regions, and performing attenuation correction on the features using the cumulative attenuation factor to calculate a corrected feature.

A program for operating an ultrasound observation apparatus according to claim 10 is provided. The ultrasound observation apparatus is configured to generate an ultrasound image based on an ultrasound signal acquired by an ultrasound probe, the ultrasound probe having an ultrasound transducer configured to transmit ultrasound to an observation target and receive the ultrasound reflected from the observation target. The program causes the ultrasound observation apparatus to execute: a frequency analysis step of, by a frequency analysis unit, analyzing a frequency of the ultrasound signal to calculate a plurality of frequency spectra in accordance with reception depths and receiving directions of the ultrasound signal; a feature calculation step of, by a feature calculation unit, calculating features of the plurality of frequency spectra; an attenuation factor setting step of, by an attenuation factor setting unit, using each of a plurality of attenuation factor candidate values per unit length and per unit frequency that give different attenuation characteristics when the ultrasound propagates through the observation target, in each of divided regions obtained by dividing the ultrasound image into a plurality of regions, to perform attenuation correction on the features of the frequency spectra for removing an influence of the ultrasound, and thereby calculating a preliminarily corrected feature of each of the frequency spectra for each of the attenuation factor candidate values, and setting an optimum attenuation factor for the observation target among the plurality of attenuation factor candidate values based on a calculation result; and a feature correction step of, by a feature correction unit, calculating a cumulative attenuation factor per unit frequency at a sampling point by using an optimum attenuation factor of a divided region present between a surface of the ultrasound transducer and the sampling point among optimum attenuation factors set respectively for the divided regions, and performing attenuation correction on the features using the cumulative attenuation factor to calculate a corrected feature.

Advantageous Effects of Invention

According to the present invention, a preliminarily corrected feature of a frequency spectrum in accordance with a plurality of attenuation factor candidate values per unit length and per unit frequency that give different attenuation characteristics is calculated in each of divided regions obtained by dividing an ultrasound image into a plurality of regions. An optimum attenuation factor for an observation target is set for each of the divided regions among the plurality of attenuation factor candidate values based on the calculation result. An optimum attenuation factor of a divided region present between a surface of an ultrasound transducer and a sampling point is used to calculate a cumulative attenuation factor per unit frequency at the sampling point. Attenuation correction is performed on a feature using the cumulative attenuation factor to calculate a corrected feature. Hence, even if attenuation factors of the observation target are nonuniform, the corrected feature can be calculated in view of the non-uniformity. Therefore, according to the present invention, it is possible to accurately identify tissue characteristics of the observation target with the nonuniform attenuation factors.

Brief Description of Drawings

FIG. 1 is a block diagram illustrating a functional configuration of an ultrasound diagnosis system including an ultrasound observation apparatus according to an embodiment of the present invention.
FIG. 2 is a diagram illustrating the relationship between the reception depth and an amplification factor in an amplification process that is performed by a signal amplification unit of the ultrasound observation apparatus according to the embodiment of the present invention.
FIG. 3 is a diagram illustrating the relationship between the reception depth and the amplification factor in an amplification correction process that is performed by an amplification correction unit of the ultrasound observation apparatus according to the embodiment of the present invention.
FIG. 4 is a diagram schematically illustrating data arrangement in one sound ray of an ultrasound signal.
FIG. 5 is a diagram illustrating an example of a frequency spectrum calculated by a frequency analysis unit of the ultrasound observation apparatus according to the embodiment of the present invention.
FIG. 6 is a diagram schematically illustrating an example of the setting of divided regions in an ultrasound image.
FIG. 7 is a diagram illustrating a straight line having, as parameters, preliminarily corrected features corrected by an attenuation factor setting unit of the ultrasound observation apparatus according to the embodiment of the present invention.
FIG. 8 is a diagram schematically illustrating an example of distributions of preliminarily corrected features that have been corrected for attenuation for the same observation target based respectively on two different attenuation factor candidate values.
FIG. 9 is a flowchart illustrating an overview of a process that is performed by the ultrasound observation apparatus according to the embodiment of the present invention.
FIG. 10 is a flowchart illustrating an overview of a process that is executed by the frequency analysis unit of the ultrasound observation apparatus according to the embodiment of the present invention.
FIG. 11 is a diagram illustrating an overview of a process that is performed by the attenuation factor setting unit of the ultrasound observation apparatus according to the embodiment of the present invention.
FIG. 12 is a diagram illustrating another example of the setting of divided regions in an ultrasound image.
FIG. 13 is a diagram illustrating an overview of another method for the setting of an optimum attenuation factor that is performed by the attenuation factor setting unit of the ultrasound observation apparatus according to the embodiment of the present invention.

Description of Embodiments

Modes for carrying out the present invention (hereinafter referred to as the "embodiment(s)") will be described below with reference to the accompanying drawings.

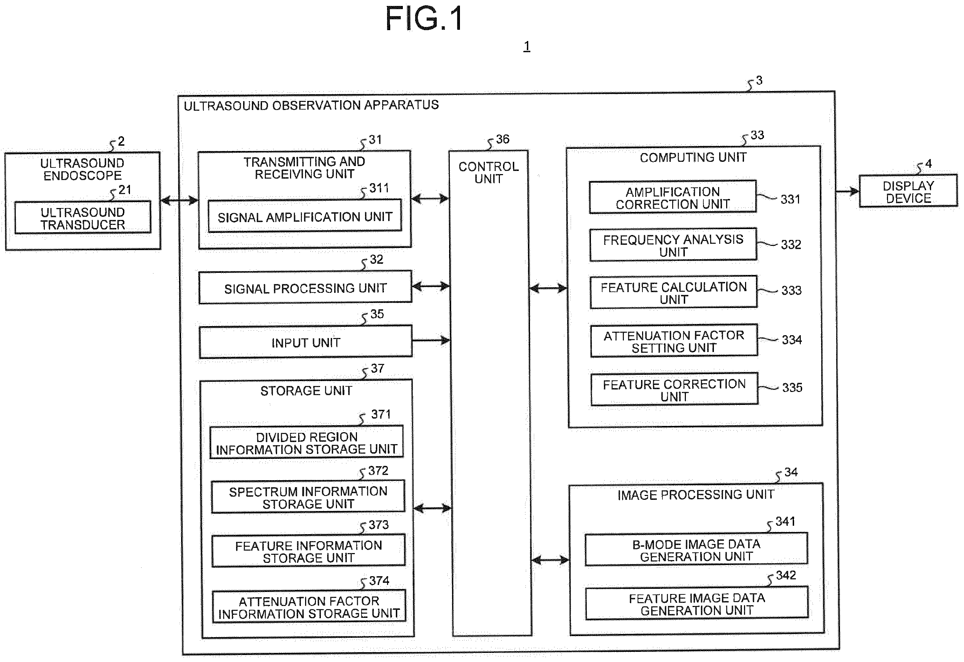

FIG. 1 is a block diagram illustrating a functional configuration of an ultrasound diagnosis system including an ultrasound observation apparatus according to an embodiment of the present invention. An ultrasound diagnosis system 1 illustrated in FIG. 1 includes an ultrasound endoscope 2 that transmits ultrasound to a subject as an observation target and receives the ultrasound reflected from the subject, an ultrasound observation apparatus 3 that generates an ultrasound image based on the ultrasound signal acquired by the ultrasound endoscope 2, and a display device 4 that displays the ultrasound image generated by the ultrasound observation apparatus 3.
The ultrasound endoscope 2 includes, in its distal end portion, an ultrasound transducer 21 that converts an electric pulse signal received from the ultrasound observation apparatus 3 into an ultrasound pulse (acoustic pulse) to apply the ultrasound pulse to the subject, and also converts an ultrasound echo reflected from the subject into an electric echo signal expressed as a voltage change to output the echo signal. The ultrasound transducer 21 can be any of a convex transducer, a linear transducer, and a radial transducer. The ultrasound endoscope 2 may be one that scans mechanically with the ultrasound transducer 21, or one that is provided with a plurality of elements in array form as the ultrasound transducer 21 to electronically switches the elements for transmission and reception and delay the transmission and reception by the elements for the purpose of scanning electronically. The ultrasound transducer 21 shall hereinafter be a linear transducer in the embodiment for explanation purposes.

The ultrasound endoscope 2 generally includes an imaging optical system and an imaging element. The ultrasound endoscope 2 is inserted into the digestive tract (esophagus, stomach, duodenum, or large intestine) or respiratory organ (trachea or bronchus) of the subject to enable the capturing of the digestive tract or respiratory organ, and its surrounding organ (such as pancreas, gallbladder, bile duct, biliary tract, lymph node, mediastinal organ, or blood vessel). Moreover, the ultrasound endoscope 2 includes a light guide that guides illumination light that is applied to the subject upon imaging. The light guide includes a distal end portion that reaches a distal end of an insertion portion into the subject of the ultrasound endoscope 2, and a proximal end portion connected to a light source device that emits the illumination light.

The ultrasound observation apparatus 3 includes: a transmitting and receiving unit 31 that is electrically connected to the ultrasound endoscope 2 to transmit a transmission signal (pulse signal) including a high voltage pulse to the ultrasound transducer 21 based on a predetermined waveform and transmission timing, and also receive an echo signal being an electric reception signal from the ultrasound transducer 21, and accordingly to generate and output digital radio frequency (RF: Radio Frequency) signal data (hereinafter referred to as RF data); a signal processing unit 32 that generates digital reception data for B-mode based on the RF data received from the transmitting and receiving unit 31; a computing unit 33 that performs a predetermined computation on the RF data received from the transmitting and receiving unit 31; an image processing unit 34 that generates various kinds of image data; an input unit 35 that is realized by user interfaces such as a keyboard, mouse, and touchscreen, and accepts various information inputs; a control unit 36 that controls the entire ultrasound diagnosis system 1; and a storage unit 37 that stores various kinds of information necessary for the operation of the ultrasound observation apparatus 3.

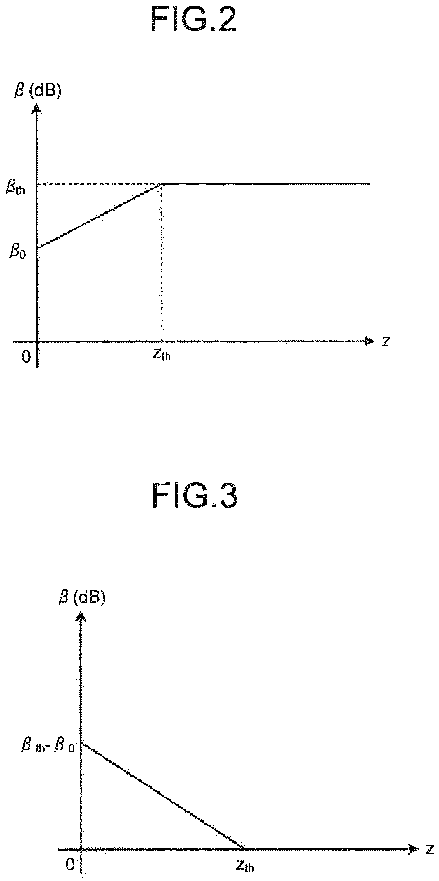

The transmitting and receiving unit 31 includes a signal amplification unit 311 that amplifies an echo signal. The signal amplification unit 311 performs STC (Sensitivity Time Control) correction that amplifies an echo signal at a higher amplification factor as the reception depth of the echo signal is increased. FIG. 2 is a diagram illustrating the relationship between the reception depth and the amplification factor in the amplification process that is performed by the signal amplification unit 311. The reception depth $z$ illustrated in FIG. 2 is a quantity calculated based on the time elapsed from the start of reception of ultrasound. As illustrated in FIG. 2, an amplification factor $\beta$ (dB) is increased linearly from $\beta_0$ to $\beta_{th}$ ($> \beta_0$) with increasing reception depth $z$ if the reception depth $z$ is less than a threshold $z_{th}$. Moreover, the amplification factor $\beta$ (dB) takes a constant value $\beta_{th}$ if the reception depth $z$ is equal to or more than the threshold $z_{th}$. The value of the threshold $z_{th}$ is a value that results in the attenuation of most of an ultrasound signal received from an observation target and predominant noise. More generally, the amplification factor $\beta$ is simply required to increase monotonously with increasing reception depth $z$ if the reception depth $z$ is less than the threshold $z_{th}$. The relationship illustrated in FIG. 2 is stored in the storage unit 37 in advance.

After performing processing such as filtering on the echo signal amplified by the signal amplification unit 311, the transmitting and receiving unit 31 performs A/D conversion to generate RF data in the time domain and outputs the RF data to the signal processing unit 32 and the computing unit 33. If the ultrasound endoscope 2 is configured to scan electronically with the ultrasound transducer 21 provided with a plurality of elements in array form, the transmitting and receiving unit 31 has a multi-channel circuit for beam synthesis corresponding to the plurality of elements.

A frequency band of the pulse signal transmitted by the transmitting and receiving unit 31 is desirably a wide band that covers most of a linear response frequency band of electroacoustic conversion from a pulse signal to an ultrasound pulse in the ultrasound transducer 21. Moreover, various processing frequency bands of the echo signal in the signal amplification unit 311 is desirably a wide band that covers most of a linear response frequency band of electroacoustic conversion from an ultrasound echo to an echo signal by the ultrasound transducer 21. Consequently, it becomes possible to accurately make an approximation in executing a frequency spectrum approximation process, which will be described below.

The transmitting and receiving unit 31 also has a function of transmitting various control signals output by the control unit 36 to the ultrasound endoscope 2 and also receiving various kinds of information including an ID for identification from the ultrasound endoscope 2 to transmit the information to the control unit 36.

The signal processing unit 32 performs known processing such as band-pass filtering, envelope detection, and logarithmic conversion on the RF data, and generates digital reception data for B-mode. In the logarithmic conversion, a decibel value representation is used taking the common logarithm of a quantity obtained by dividing the RF data by a reference voltage. The signal processing unit 32 outputs the generated reception data for B-mode to the image processing unit 34. The signal processing unit 32 is realized by a Central Processing Unit (CPU), various arithmetic circuits, and the like.

The computing unit 33 includes: an amplification correction unit 331 that performs amplification correction on the RF data output by the transmitting and receiving unit 31 to make the amplification factor constant irrespective of the reception depth; a frequency analysis unit 332 that performs the fast Fourier transform (FFT) on the RF data on which the amplification correction has been performed for a frequency analysis and, accordingly, to calculate a plurality of frequency spectra in accordance with the reception depths and receiving directions of an ultrasound signal; a feature calculation unit 333 that calculates features of each frequency spectrum; an attenuation factor setting unit 334 that, in each divided region obtained by dividing an ultrasound image into a plurality of regions, uses each of a plurality of attenuation factor candidate values per unit length and per unit frequency that gives different attenuation characteristics from each other of when ultrasound propagates through an observation target to perform attenuation correction that removes the influence of the ultrasound on the features of each frequency spectrum, and accordingly, to calculate preliminarily corrected features of each frequency spectrum for each attenuation factor candidate value and set an optimum attenuation factor for the observation target from the plurality of attenuation factor candidate values based on the calculation result; and a feature correction unit 335 that uses an optimum attenuation factor of a divided region present between the surface of the ultrasound transducer and a sampling point among the optimum attenuation factors set respectively for the divided regions by the attenuation factor setting unit 334 to calculate a cumulative attenuation factor per unit frequency at the sampling point, and performs attenuation correction on the features with the cumulative attenuation factor to calculate corrected features. The computing unit 33 is realized by using a CPU, various arithmetic circuits, and the like.

FIG. 3 is a diagram illustrating the relationship between the reception depth and the amplification factor in the amplification correction process that is performed by the amplification correction unit 331. As illustrated in FIG. 3, the amplification factor $\beta$ (dB) in the amplification process that is performed by the amplification correction unit 331 takes a maximum value $\beta_{th}$ - $\beta_0$ when the reception depth z is zero, reduces linearly until the reception depth z reaches from zero to the threshold $z_{th}$, and is zero when the reception depth z is equal to or more than the threshold $z_{th}$. The relationship illustrated in FIG. 3 is stored in the storage unit 37 in advance. The amplification correction unit 331 performs amplification correction on the digital RF data based on the relationship illustrated in FIG. 3. Accordingly, it is possible to cancel the influence of the STC correction in the signal amplification unit 311 and output a signal with a constant amplification factor $\beta_{th}$. The relationship between the reception depth z and the amplification factor $\beta$ in the amplification correction process that is performed by the amplification correction unit 331 is naturally different depending on the relationship between the reception depth and the amplification factor in the amplification process that is performed by the signal amplification unit 311.

The reason why such amplification correction is performed is hereinafter described. The STC correction is a correction process that amplifies the amplitude of an analog signal waveform uniformly over the entire frequency band and with the amplification factor that increases monotonously with respect to the depth, and accordingly removes the influence of attenuation from the amplitude of the analog signal waveform. Hence, if a B-mode image is generated which displays the amplitude of an echo signal after conversion into luminance, and if uniform tissue is scanned, the luminance value becomes constant irrespective of the depth due to the performance of the STC correction. In other words, it is possible to obtain an effect that removes the influence of attenuation from the luminance value of the B-mode image.

On the other hand, if the calculation and analysis result of a frequency spectrum of ultrasound is used as in the embodiment, there is a problem that the influence of attenuation associated with the propagation of ultrasound cannot always be removed accurately even in the STC correction. This is because the amount of attenuation is generally different depending on the frequency (refer to equation (1) described below), but the amplification factor of the STC correction changes according only to the distance and is not frequency dependent.

In order to solve the above-mentioned problem, it is conceivable that a reception signal on which the STC correction has been performed is output when a B-mode image is generated, while, at the time of generating an image based on a frequency spectrum, a new transmission different from the transmission for generating the B-mode image is performed to output a reception signal on which the STC correction has not been performed. However, in this case, there is a problem that the frame rate of image data generated based on the reception signal is reduced.

Hence, in the embodiment, the amplification correction unit 331 corrects the amplification factor for a signal on which the STC correction has been performed for a B-mode image in order to remove the influence of the STC correction while maintaining the frame rate of image data generated.

The frequency analysis unit 332 samples RF data (line data) of each sound ray corrected for amplification by the amplification correction unit 331 at predetermined time intervals, and generates sample data. The frequency analysis unit 332 performs the FFT process on sample data groups to calculate a frequency spectrum at a plurality of points (data positions) on the RF data.

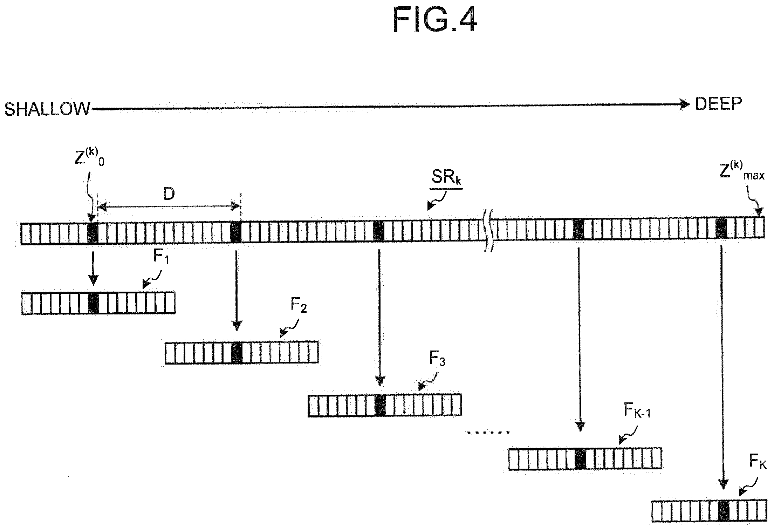

FIG. 4 is a diagram schematically illustrating data arrangement in one sound ray of an ultrasound signal. In a sound ray $SR_k$ illustrated in FIG. 4, a white or black rectangle indicates data at one sample point. Moreover, in the sound ray $SR_k$, data located at a further right position is sample data from a deeper point when measurements are taken along the sound ray $SR_k$ from the ultrasound transducer 21 (refer to an arrow in FIG. 4). The sound ray $SR_k$ is discretized at

intervals of time corresponding to a sampling frequency (for example, 50 MHz) in A/D conversion performed by the transmitting and receiving unit 31. FIG. 4 illustrates a case where the position of the eighth data of the sound ray $SR_k$ of number k is set as an initial value $Z^{(k)}_0$ in the direction of the reception depth z. However, the position of the initial value can be set freely. The result of the calculation made by the frequency analysis unit 332 is obtained in a complex number and stored in the storage unit 37.

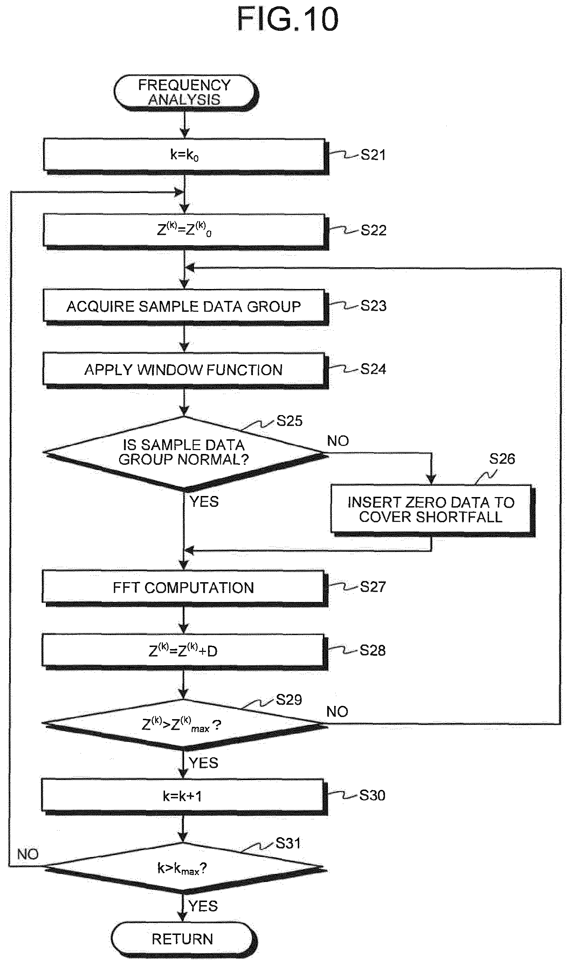

A data group $F_j$ (j = 1, 2, ..., K) illustrated in FIG. 4 is a sample data group targeted for the FFT process. The sample data group is generally required to have the number of pieces of data being a power of 2 for the FFT process. In this sense, the sample data group $F_j$ (j = 1, 2, ..., K-1) includes 16 (= $2^4$) pieces of data and is a normal data group, while a sample data group $F_k$ includes 12 pieces of data and therefore is an abnormal data group. When the FFT process is performed on the abnormal data group, a process is performed in which zero data covering the shortfall is inserted to generate a normal sample data group. In this regard, a detailed description is given when the process of the frequency analysis unit 332 is described (refer to FIG. 10).

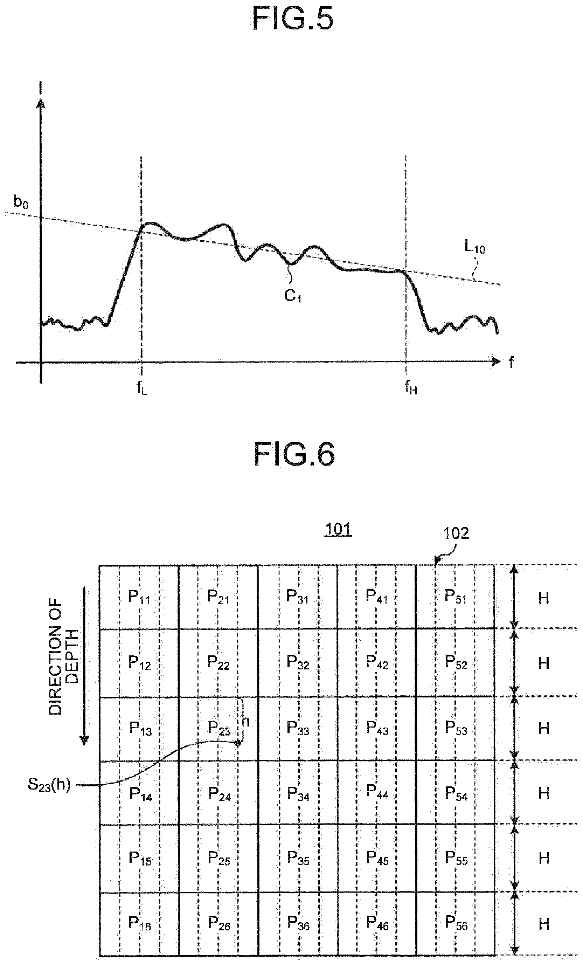

FIG. 5 is a diagram illustrating an example of the frequency spectrum calculated by the frequency analysis unit 332. "Frequency spectrum" here indicates the "distribution of frequencies at an intensity at some reception depth z" obtained by performing the FFT process on a sample data group. Moreover, "intensity" here indicates any of, for example, parameters such as the voltage of an echo signal, the power of the echo signal, the sound pressure of the ultrasound echo, and the sound energy of the ultrasound echo, the amplitudes and time integrated values of these parameters, and their combinations.

In FIG. 5, the horizontal axis represents a frequency f. Moreover, in FIG. 5, the vertical axis represents the common logarithm (expressed in decibels) of a quantity obtained by dividing an intensity $I_0$ by a reference intensity $I_c$ (constant), $I = 10 \log_{10} (I_0 / I_c)$. In FIG. 5, the reception depth z is constant. A straight line $L_{10}$ illustrated in FIG. 5 is described below. In the embodiment, a curve and a straight line each include a set of discrete points.

In a frequency spectrum $C_1$ illustrated in FIG. 5, a lower-limit frequency $f_L$ and an upper-limit frequency $f_H$ of a frequency band, which is used for a subsequent computation, are parameters that are determined based on the frequency band of the ultrasound transducer 21, the frequency band of a pulse signal transmitted by the transmitting and receiving unit 31, and the like. For example, $f_L$ = 3 MHz, and $f_H$ = 10 MHz. In FIG. 5, the frequency band determined by the lower-limit frequency $f_L$ and the upper-limit frequency $f_H$ is hereinafter referred to as the "frequency band U."

If the observation target is living tissue, the frequency spectrum generally indicates a different tendency depending on the characteristics of the living tissue that is scanned with ultrasound. This is because the frequency spectrum has a correlation to the size, number density, acoustic impedance, and the like of a scatterer that scatters ultrasound. The "characteristics of the living tissue" herein indicates, for example, a malignant tumor (cancer), a benign tumor, an endocrine tumor, a mucinous tumor, normal tissue, a cyst, and a vas.

The feature calculation unit 333 performs a regression analysis on a frequency spectrum in a predetermined frequency band and approximates the frequency spectrum by a linear expression, thereby calculating features that characterize the linear expression approximated. For example, in a case of the frequency spectrum $C_1$ illustrated in FIG. 5, the feature calculation unit 333 performs a regression analysis in the frequency band U to obtain the approximate straight line $L_{10}$. Suppose that the approximate straight line $L_{10}$ is expressed below in a linear expression of the frequency f, $I = a_0 f + b_0$. The feature calculation unit 333 calculates, as the features corresponding to the straight line $L_{10}$, a slope $a_0$, an intercept $b_0$, and a midband fit $c_0 = a_0 f_M + b_0$ that is a value of the intensity I at a center frequency in the frequency band U, $f_M = (f_s + f_e)/2$. The feature calculation unit 333 may approximate the frequency spectrum by a quadratic or higher order polynomial using a regression analysis.

Among the three features before correction, the slope $a_0$ is considered to have a correlation with the size of the scatterer of ultrasound, and generally have a smaller value as the scatterer is increased in size. Moreover, the intercept $b_0$ has a correlation with scatterer size, difference in acoustic impedance, scatterer number density (concentration), and the like. Specifically, the intercept $b_0$ is considered to have a larger value as the scatterer is increased in size, have a larger value as the difference in acoustic impedance is increased, and have a larger value as the number density of the scatterer is increased. The midband fit $c_0$ is an indirect parameter that is derived from the slope $a_0$ and the intercept $b_0$, and gives the intensity of a spectrum at the center of the effective frequency band. Hence, the midband fit $c_0$ is considered to have some correlation with the luminance of a B-mode image in addition to the scatterer size, the difference in acoustic impedance, and the scatterer number density.

The attenuation factor setting unit 334 performs attenuation correction on the features in each divided region obtained by dividing an ultrasound image into a plurality of regions, using the attenuation factor that gives the amount of attenuation of ultrasound per unit length and per unit frequency. FIG. 6 is a diagram schematically illustrating an example of the setting of divided regions in an ultrasound image. FIG. 6 illustrates a case where an ultrasound image 101 that forms a rectangular shape is divided into 30 divided regions $P_{ij}$ (i = 1 to 5, j = 1 to 6). Broken lines of FIG. 6 indicate sound rays. In the case of FIG. 6, all of the divided regions $P_{ij}$ have the same area. Hence, a depth H in the direction of depth from a surface position 102 of the ultrasound transducer 21 in the divided region $P_{ij}$ (hereinafter referred to as the height H of the divided region $P_{ij}$) is constant. Information related to the divided region $P_{ij}$ is stored in a divided region information storage unit 371 of the storage unit 37. The number of sound rays is only a mere example, and is not limited to the case illustrated in FIG. 6. Moreover, FIG. 6 illustrates an ultrasound image of a case where the ultrasound transducer 21 is a linear transducer, and accordingly the ultrasound image forms a rectangular shape. However, it is needless to say that the outer shape of the ultrasound image is different if the ultrasound transducer 21 is of another type.

The amount of attenuation A (f, z) of ultrasound is attenuation that occurs while ultrasound travels between the reception depth 0 and the reception depth z, and is defined as an intensity change (a difference expressed in decibels) before and after the round trip. The amount of attenuation A (f, z) is empirically known to be proportional to the frequency in uniform tissue, and is expressed by the following equation (1):

$$A(f, z) = 2\alpha z f \qquad\qquad (1).$$

Here, the proportionality constant $\alpha$ is a quantity called the attenuation factor, and gives the amount of attenuation of ultrasound per unit length and per unit frequency. Moreover, z is the reception depth of ultrasound, and f is the frequency. If the observation target is a living body, a specific value of the attenuation factor $\alpha$ is determined in accordance with the region of the living body. The unit of the attenuation factor $\alpha$ is, for example, dB/cm/MHz.

The attenuation factor setting unit 334 sets an optimum attenuation factor from a plurality of attenuation factor candidate values. At this point in time, the attenuation factor setting unit 334 performs attenuation correction on the features (the slope $a_0$, the intercept $b_0$, and the midband fit $c_0$) calculated by the feature calculation unit 333 in accordance with equations (2) to (4) illustrated below, using the attenuation factor candidate value $\alpha$, to calculate preliminarily corrected features a, b, and c.

$$a = a_0 + 2\alpha z \qquad\qquad (2)$$

$$b = b_0 \qquad\qquad (3)$$

$$c = c_0 + A(f_M, z) = c_0 + 2\alpha z f_M \ (= af_M + b) \qquad (4)$$

As is clear from equations (2) and (4), the attenuation factor setting unit 334 makes corrections in which the amount of correction is increased with increasing reception depth z of ultrasound. Moreover, according to equation (3), the correction related to the intercept is the identity transformation. This is because the intercept is a frequency component corresponding to a frequency of zero (Hz) and is not influenced by attenuation.

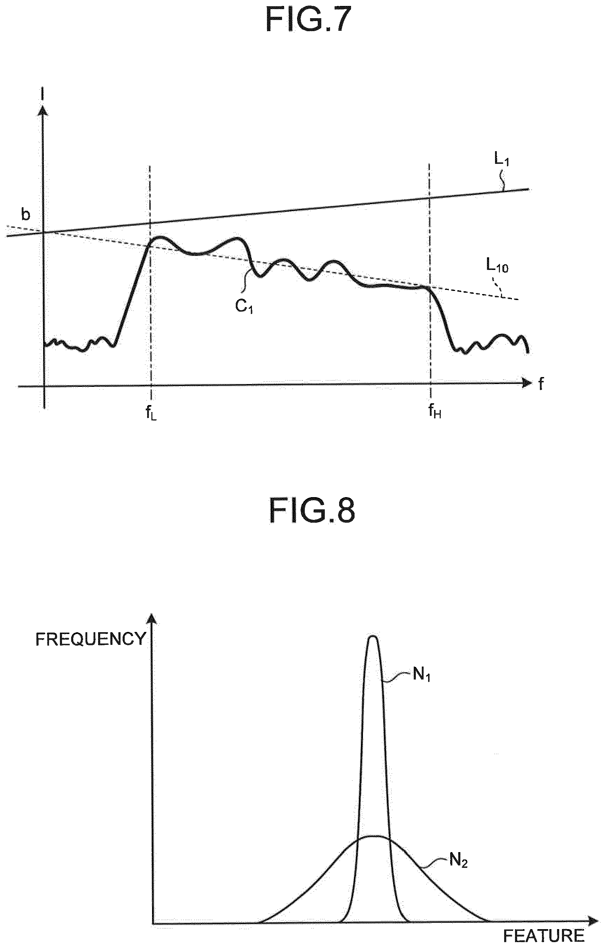

FIG. 7 is a diagram illustrating a straight line having, as parameters, the preliminarily corrected features a, b, and c corrected as the parameters by the attenuation factor setting unit 334. An equation of a straight line $L_1$ is expressed as

$$I = af + b = (a_0 + 2\alpha z)f + b_0 \qquad\qquad (5).$$

As is clear from equation (5), the straight line $L_1$ is larger in slope (a > $a_0$) and the same in intercept (b = $b_0$) compared to the straight line $L_{10}$ before attenuation correction.

The attenuation factor setting unit 334 sets an attenuation factor candidate value having minimal statistical dispersion of the preliminarily corrected feature calculated for each attenuation factor candidate value, as the optimum attenuation factor, in each divided region. In the embodiment, the variance is applied as a quantity indicating statistical dispersion. In this case, the attenuation factor setting unit 334 sets an attenuation factor candidate value having a minimum variance as the optimum attenuation factor. Two of the above-mentioned preliminarily corrected features a, b, and c are independent. In addition, the preliminarily corrected feature b is not dependent on the attenuation factor. Therefore, if the optimum attenuation factor is set for the preliminarily corrected features a, c, the attenuation factor setting unit 334 is simply required to calculate the variance of one of the preliminarily corrected features a and c.

However, it is more preferable that, the variance of the preliminarily corrected feature a be applied if the attenuation factor setting unit 334 sets the optimum attenuation factor using the preliminarily corrected feature a, , and the variance of the preliminarily corrected feature c be applied if setting the optimum attenuation factor using the preliminarily corrected feature c. This is because equation (1) that gives the amount of attenuation A (f, z) is simply ideal, but the following equation (6) is more appropriate in practice.

$$A(f, z) = 2\alpha z f + 2\alpha_1 z \qquad\qquad (6)$$

$\alpha_1$ of the second term on the right-hand side of equation (6) is a coefficient indicating the magnitude of a change in signal intensity in proportion to the reception depth z of ultrasound, and is a coefficient indicating a change in signal intensity that occurs due to nonuniformity of tissue of the observation target, a change in the number of channels upon beam synthesis, and the like. Because of the second term on the right-hand side of equation (6), when the optimum attenuation factor is set using the preliminarily corrected feature c, it is possible to correct attenuation more accurately with the application of the variance of the preliminarily corrected feature c (refer to equation (4)). On the other hand, if the optimum attenuation factor is set using the preliminarily corrected feature a being a coefficient that is proportional to the frequency f, it is possible to remove the influence of the second term on the right-hand side of equation (6) and correct attenuation more accurately with the application of the variance of the preliminarily corrected feature a.

The reason why the optimum attenuation factor can be set based on statistical dispersion is hereinafter described. It is considered that, if the optimum attenuation factor is applied to an observation target, a feature converges to a value specific to the observation target irrespective of the distance between the observation target and the ultrasound transducer 21, and statistical dispersion is reduced. On the other hand, it is considered that, if an attenuation factor candidate value that is not fit for the observation target is set as the optimum attenuation factor, the attenuation correction is excessive or insufficient. Accordingly, the feature varies according to the distance to the ultrasound transducer 21, and the statistical dispersion of the feature is increased. Therefore, it can be said that an attenuation factor candidate value having minimal statistical dispersion is the optimum attenuation factor for the observation target.

FIG. 8 is a diagram schematically illustrating an example of distributions of preliminarily corrected features that have been corrected for attenuation for the same observation target based respectively on two different attenuation factor candidate values. In FIG. 8, the horizontal axis indicates the preliminarily corrected feature, and the vertical axis indicates the frequency. Two distribution curves $N_1$ and $N_2$ illustrated in FIG. 8 have the same sum of frequencies. In the case illustrated in FIG. 8, the distribution curve $N_1$ has less statistical dispersion (less variance) of the feature than the distribution curve $N_2$, and has a crest that forms a steep shape. Therefore, if setting the optimum attenuation factor from the two attenuation factor candidate values corresponding to the two distribution curves $N_1$ and $N_2$, the attenuation factor setting unit 334 sets the attenuation factor candidate value corresponding to the distribution curve $N_1$ as the optimum attenuation factor.

The feature correction unit 335 calculates a cumulative attenuation factor per unit frequency at a sampling point (hereinafter simply referred to as the cumulative attenuation factor) using an optimum attenuation factor of each divided region, and performs attenuation correction on the features using the cumulative attenuation factor. The cumulative attenuation factor at a given sampling point is calculated using the distance from the surface of the ultrasound transducer 21 and an optimum attenuation factor of a divided region present in between with the surface. A cumulative attenuation factor $\gamma_{IJ}(h)$ at a sampling point $S_{IJ}(h)$ whose distance from a boundary on a side close to the ultrasound transducer 21 is h ($0 < h \leq H$) in a divided region $P_{IJ}$ ($1 \leq I \leq i_{max}$, $1 \leq J \leq j_{max}$; I and J are constants) is expressed as:

$$\gamma_{IJ}(h) = [\Sigma_{j=1,2,\ldots,J-1} (2H \cdot \alpha(P_{Ij}))] + 2h \cdot \alpha(P_{IJ}) \quad (7).$$

Here, $\Sigma_{j=1,2,\ldots,J-1}$ indicates the sum over j = 1 to J-1, and $\alpha(P_{Ij})$ represents the optimum attenuation factor in the divided region $P_{Ij}$. The first term on the right-hand side of equation (7) is the sum of those obtained by multiplying the round-trip distance 2H of ultrasound of each divided region by the optimum attenuation factor $\alpha(P_{IJ})$. The second term on the right-hand side of equation (7) is obtained by multiplying the round-trip distance 2h of ultrasound to the sampling point $S_{IJ}(h)$ in the divided region $P_{IJ}$ by the optimum attenuation factor $\alpha(P_{IJ})$ in the divided region $P_{IJ}$. In this manner, the feature correction unit 335 accumulates the attenuation factors from the surface of the ultrasound transducer 21 to calculate the cumulative attenuation factor $\gamma_{IJ}(h)$. Assuming that the unit of the optimum attenuation factor is dB/cm/MHz, the unit of the cumulative attenuation factor is dB/MHz.

For example, FIG. 6 illustrates a case where I = 2 and J = 3 (where $i_{max}$ = 5 and $j_{max}$ = 6) in equation (7). In this case, from equation (7), a cumulative attenuation factor $\gamma_{23}(h)$ at a sampling point $S_{23}(h)$ is

$$\gamma_{23}(h) = 2H \cdot \alpha(P_{21}) + 2H \cdot \alpha(P_{22}) + 2h \cdot \alpha(P_{23}) \ldots (8).$$

The feature correction unit 335 performs attenuation correction on the features at the sampling point $S_{IJ}(h)$ using the cumulative attenuation factor $\gamma_{IJ}(h)$ as follows:

$$a_{IJ}(h) = a_0 + 2\gamma_{IJ}(h) \tag{9}$$

$$b_{IJ}(h) = b_0 \tag{10}$$

$$c_{IJ}(h) = c_0 + 2f_M\gamma_{IJ}(h) \tag{11}$$

The image processing unit 34 includes a B-mode image data generation unit 341 that generates B-mode image data being an ultrasound image that displays the amplitude of an echo signal after conversion into luminance, and a feature image data generation unit 342 that generates feature image data that shows information on the features calculated by the feature calculation unit 333.

The B-mode image data generation unit 341 performs signal processing on the reception data for B-mode received from the signal processing unit 32, using known technologies such as gain processing and contrast processing, and also, for example, reduces data in accordance with the data step width determined according to the image display range of the display device 4. Accordingly, the B-mode image data generation unit 341 generates B-mode image data. A B-mode image is a grayscale image where the values of R (red), G (green), and B (blue), which are variables when the RGB color system is adopted as the color space, are made equal.

The B-mode image data generation unit 341 performs coordinate conversion on the reception data for B-mode for rearrangement so as to spatially represent scanning ranges correctly, and then performs interpolation between the reception data for B-mode. Accordingly, the B-mode image data generation unit 341 bridges gaps between the reception data for B-mode, and generates B-mode image data. The B-mode image data generation unit 341 outputs the generated B-mode image data to the feature image data generation unit 342.

The feature image data generation unit 342 superimposes visual information related to the feature(s) calculated by the feature calculation unit 333 on each pixel of an image of the B-mode image data, and accordingly generates feature image data. The feature image data generation unit 342 assigns visual information corresponding to the feature(s) of a frequency spectrum calculated from, for example, one amplification data group $F_j$ (j = 1, 2, ..., K) illustrated in FIG. 4 to a pixel region corresponding to the data amount of the amplification data group $F_j$. The feature image data generation unit 342 associates hue as the visual information with any of, for example, the above-mentioned slope, intercept, and midband fit to generate feature image data. The feature image data generation unit 342 may associate hue with one of two features selected from the slope, intercept, and midband fit, and associate contrast with the other, to generate feature image data. Examples of the visual information on the feature(s) include variables of a color space forming a predetermined color system such as saturation, luminance value, R (red), G (green), and B (blue), in addition to hue and contrast (brightness).

The control unit 36 is realized by a Central Processing Unit (CPU) having a computation and control function, various arithmetic circuits, and the like. The control unit 36 reads information retained and stored in the storage unit 37 from the storage unit 37, executes various computation processes related to a method for operating the ultrasound observation apparatus 3, and accordingly integrally controls the ultrasound observation apparatus 3. The control unit 36 may share a common CPU and the like with the signal processing unit 32 and the computing unit 33.

The storage unit 37 includes the divided region information storage unit 371 that stores information on the divided regions, a spectrum information storage unit 372 that stores information on the frequency spectra calculated by the frequency analysis unit 332 together with the reception depths and the receiving directions, a feature information storage unit 373 that stores information on the features calculated by the feature calculation unit 333 and the corrected features corrected by the feature correction unit 335, and an attenuation factor information storage unit 374 that stores information on an optimum attenuation factor set for each divided region by the attenuation factor setting unit 334 and a cumulative attenuation factor of each sampling point calculated by the feature correction unit 335.

In addition to the above information, the storage unit 37 stores, for example, information necessary for the amplification process (the relationship between the amplification factor and the reception depth illustrated in FIG. 2), information necessary for the amplification correction process (the relationship between the attenuation factor and the reception depth illustrated in FIG. 3), information necessary for the attenuation correction process (refer to equation (1)), and information on a window function (such as Hamming, Hanning, or Blackman) necessary for the frequency analysis process.

Moreover, the storage unit 37 stores various programs including an operation program for executing the method for operating the ultrasound observation apparatus 3. The operation program can also be recorded in computer-readable recording media such as hard disks, flash memories, CD-ROMs, DVD-ROMs, and flexible disks to be put into general circulation. The above-mentioned various programs can also be acquired by being downloaded via communication networks. The communication networks here are realized by, for example, an existing public network, Local Area Network (LAN), and Wide Area Network (WAN) irrespective of wired or wireless.

The storage unit 37 having the above configuration is realized by, for example, a Read Only Memory (ROM) in which various programs and the like are preinstalled, and a Random Access Memory (RAM) in which computational parameters, data, and the like of various processes are stored.

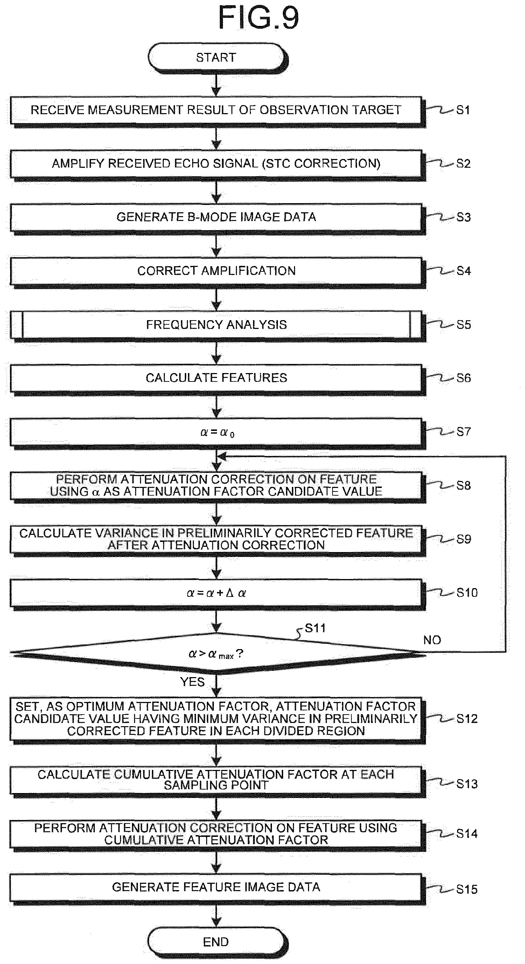

FIG. 9 is a flowchart illustrating an overview of a process that is performed by the ultrasound observation apparatus 3 having the above configuration. Specifically, FIG. 9 is a flowchart illustrating an overview of a process after the ultrasound observation apparatus 3 receives an echo signal from the ultrasound endoscope 2. The process that is performed by the ultrasound observation apparatus 3 is described hereinafter with reference to FIG. 9. First, the ultrasound observation apparatus 3 receives, from the ultrasound endoscope 2, an echo signal as a measurement result of the observation target by the ultrasound transducer 21 (Step S1).

The signal amplification unit 311, which has received the echo signal from the ultrasound transducer 21, amplifies the echo signal (Step S2). The signal amplification unit 311 performs an amplification (the STC correction) on the echo signal based on, for example, the relationship between the amplification factor and the reception depth illustrated in FIG. 2.

Next, the B-mode image data generation unit 341 generates B-mode image data using the echo signal amplified by the signal amplification unit 311, and outputs the B-mode image data to the display device 4 (Step S3). The display device 4, which has received the B-mode image data, displays a B-mode image corresponding to the B-mode image data.

The amplification correction unit 331 performs amplification correction on the RF data output from the transmitting and receiving unit 31 such that the amplification factor is constant irrespective of the reception depth (Step S4). The amplification correction unit 331 performs amplification correction based on, for example, the relationship between the amplification factor and the reception depth illustrated in FIG. 3.

The frequency analysis unit 332 then performs a frequency analysis by the FFT on the RF data of each sound ray after amplification correction to calculate frequency spectra for all sample data groups, and stores them in the spectrum information storage unit 372 (Step S5). FIG. 10 is a flowchart illustrating an overview of the process that is performed by the frequency analysis unit 332 in Step S5. The details of the frequency analysis process are described hereinafter with reference to the flowchart illustrated in FIG. 10.

First, the frequency analysis unit 332 sets a counter k that identifies a sound ray targeted for analysis to $k_0$ (Step S21).

Next, the frequency analysis unit 332 sets an initial value $Z^{(k)}_0$ of a data position (corresponding to the reception depth) $Z^{(k)}$ representing a series of data groups (sample data groups) generated for FFT computation (Step S22). For example, FIG. 4 illustrates the case where the position of the eighth data of the sound ray $SR_k$ is set as an initial value $Z^{(k)}_0$ as described above.

Next, the frequency analysis unit 332 acquires the sample data groups (Step S23), and applies a window function stored in the storage unit 37 to the acquired sample data groups (Step S24). The window function is applied to the sample data groups in this manner and accordingly, it is possible to avoid the sample data groups from becoming discontinuous at their boundaries and prevent the occurrence of an artifact.

Next, the frequency analysis unit 332 determines whether or not the sample data group at the data position $Z^{(k)}$ is a normal data group (Step S25). As described with reference to FIG. 4, the sample data group needs to include the number of pieces of data being a power of 2. The number of pieces of data of a normal sample data group shall be $2^n$ (n is a positive integer) below. In the embodiment, the data position $Z^{(k)}$ is set to be located as close to the center of a sample data group to which $Z^{(k)}$ belongs as possible. Specifically, since the number of pieces of data of a sample data group is $2^n$, $Z^{(k)}$ is set at the $2^n/2$ (= $2^{n-1}$)-th position close to the center of the sample data group. In this case, that the sample data group is normal indicates that there are $2^{n-1} - 1$ (= N) pieces of data on the shallower side than the data position $Z^{(k)}$, and there are $2^{n-1}$ (= M) pieces of data on the deeper side than the data position $Z^{(k)}$. In the case of FIG. 4, the sample data group $F_j$ (j = 1, 2, ..., K - 1) is normal. FIG. 4 illustrates a case where n = 4 (N = 7, M = 8).

If the sample data group at the data position $Z^{(k)}$ is normal as the result of the determination in Step S25 (Step S25: Yes), the frequency analysis unit 332 proceeds to Step S27 described below.

If the sample data group at the data position $Z^{(k)}$ is not normal as the result of the determination in Step S25 (Step S25: No), the frequency analysis unit 332 generates a normal sample data group by inserting zero data covering the shortfall (Step S26). The sample data group determined to be not normal in Step S25 (for example, the sample data group $F_K$ of FIG. 4) is applied a window function before being added zero data. Hence, even if zero data is inserted into the sample data group, the discontinuity of data does not occur. After Step S26, the frequency analysis unit 332 proceeds to Step S27 described below.

In Step S27, the frequency analysis unit 332 performs the FFT computation using a sample data group to obtain a frequency spectrum being the amplitude-frequency distribution (Step S27).

Next, the frequency analysis unit 332 changes the data position $Z^{(k)}$ by a step width D (Step S28). The step width D is stored in the storage unit 37 in advance. FIG. 4 illustrates a case where D = 15. The step width D is desired to agree with the data step width that is used when the B-mode image data generation unit 341 generates B-mode image data. However, if the amount of computation in the frequency analysis unit 332 is desired to be reduced, a larger value than the data step width may be set as the step width D.

The frequency analysis unit 332 then determines whether or not the data position $Z^{(k)}$ is larger than a maximum value $Z^{(k)}_{max}$ of the sound ray $SR_k$ (Step S29). If the data position $Z^{(k)}$ is larger than the maximum value $Z^{(k)}_{max}$ (Step S29: Yes), the frequency analysis unit 332 increments the counter k by one (Step S30). This indicates a shift of the processing to the adjacent sound ray. On the other hand, if the data position $Z^{(k)}$ is equal to or less than the maximum value $Z^{(k)}_{max}$ (Step S29: No), the frequency analysis unit 332 returns to Step S23.

After Step S30, the frequency analysis unit 332 determines whether or not the counter k is larger than the maximum value $k_{max}$ (Step S31). If the counter k is larger than $k_{max}$ (Step S31: Yes), the frequency analysis unit 332 ends a series of the frequency analysis process steps. On the other hand, if the counter k is equal to or less than $k_{max}$ (Step S31: No), the frequency analysis unit 332 returns to Step S22. The maximum value $k_{max}$ is a value that a user such as an operator has input through the input unit 35, or a value that is preset in the storage unit 37.

In this manner, the frequency analysis unit 332 performs FFT computation multiple times on each of $(k_{max} - k_0 + 1)$ sound rays within an analysis target region. The frequency spectra obtained as the result of the FFT computation, together with the reception depths and the receiving directions, are stored in the spectrum information storage unit 372.

In the above explanation, the frequency analysis unit 332 performs the frequency analysis process on all the regions that have received the ultrasound signal. However, the input unit 35 may accept setting and input of a partial region divided by a specific depth width and sound ray width, and the frequency analysis process may be performed on only the set partial region.

Subsequent to the frequency analysis process of Step S5 described above, the feature calculation unit 333 calculates features of a frequency spectrum at a sampling point included in each divided region (Step S6). Specifically, the feature calculation unit 333 performs a regression analysis on a frequency spectrum in a predetermined frequency band to approximate the frequency spectrum by a linear expression $I = a_0 f + b_0$, and calculates the slope $a_0$, the intercept $b_0$, and the midband fit $c_0$ as the features. For example, the straight line $L_{10}$ illustrated in FIG. 5 is a regression line obtained by approximating the frequency spectrum $C_1$ in the frequency band U by the feature calculation unit 333 through a regression analysis.

The attenuation factor setting unit 334 then sets, as a predetermined initial value $\alpha_0$, the value of the attenuation factor candidate value $\alpha$ that is applied upon the performance of attenuation correction described below (Step S7). It is preferable that the initial value $\alpha_0$ should be stored in the attenuation factor information storage unit 374 in advance.

Next, the attenuation factor setting unit 334 performs attenuation correction on the features obtained by approximating each frequency spectrum by the feature calculation unit 333, using $\alpha$ as the attenuation factor candidate value, to calculate preliminarily corrected features, and stores the preliminarily corrected features together with the attenuation factor candidate value $\alpha$ in the feature information storage unit 373 (Step S8). The straight line $L_1$ illustrated in FIG. 7 is an example of a straight line obtained by the attenuation factor setting unit 334 performing the attenuation correction process.

In Step S8, the attenuation factor setting unit 334 does the calculations by substituting the data position $Z = (f_{sp} / 2v_s)Dn$ obtained using data arrangement of a sound ray of an ultrasound signal into the reception depth z in the above-mentioned equations (2) and (4). $f_{sp}$ is the sampling frequency of data, $v_s$ is the velocity of sound, D is the data step width, and n is the number of data steps from the first data of the sound ray up to the data position of an amplification data group as a process target. For example, assuming that the data sampling frequency $f_{sp}$ is 50 MHz; the velocity of sound $v_s$ is 1530 m/sec; and the step width D is 15 by adopting the data arrangement illustrated in FIG. 4, z = 0.2295 n(mm).

The attenuation factor setting unit 334 calculates the variance in a representative preliminarily corrected feature among the plurality of preliminarily corrected features obtained by performing attenuation correction on each frequency spectrum by the attenuation factor setting unit 334, associates the variance with the attenuation factor candidate value $\alpha$, and stores the variance in the feature information storage unit 373 (Step S9). For example, if the preliminarily corrected features are the slope a and the midband fit c, the attenuation factor setting unit 334 calculates the variance of one of the preliminarily corrected features a and c as described above. It is preferable in Step S9 that, the variance of the preliminarily corrected feature a in each divided region be applied if the preliminarily corrected feature a is used to generate a feature image, , and the variance of the preliminarily corrected feature c in each divided region be applied if the preliminarily corrected feature c is used to generate a feature image

The attenuation factor setting unit 334 then increments the value of the attenuation factor candidate value $\alpha$ by $\Delta\alpha$ (Step S10), and compares the magnitudes of the incremented attenuation factor candidate value $\alpha$ and a predetermined maximum value $\alpha_{max}$ (Step S11). If the attenuation factor candidate value $\alpha$ is larger than the maximum value $\alpha_{max}$ as the result of the comparison in Step S11 (Step S11: Yes), the ultrasound observation apparatus 3 proceeds to Step S12. On the other hand, if the attenuation factor candidate value $\alpha$ is equal to or less than the maximum value $\alpha_{max}$ as the result of the comparison in Step S11 (Step S11: No), the ultrasound observation apparatus 3 returns to Step S8.

In Step S12, the attenuation factor setting unit 334 refers to the variance in the preliminarily corrected feature of each attenuation factor candidate value stored in the feature information storage unit 373, for each divided region, and sets an attenuation factor candidate value having a minimum variance as the optimum attenuation factor in the divided region (Step S12).

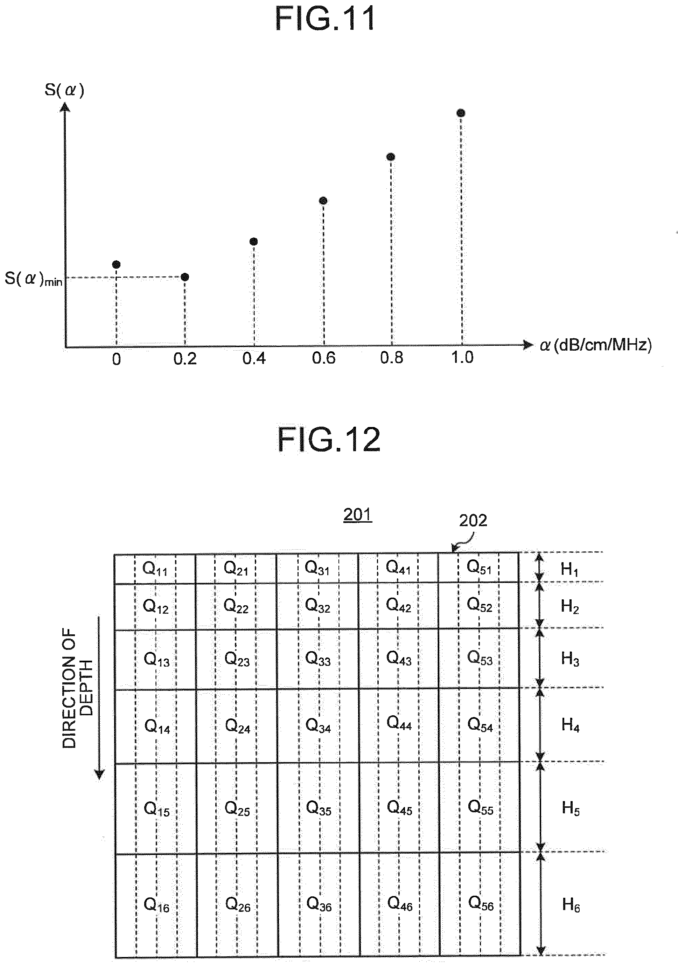

FIG. 11 is a diagram illustrating an overview of a process that is performed by the attenuation factor setting unit 334. FIG. 11 is a diagram illustrating an example of the relationship between the attenuation factor candidate value $\alpha$ and a variance S(a), where $\alpha_0$ = 0 (dB/cm/MHz), $\alpha_{max}$ = 1.0 (dB/cm/MHz), and $\Delta\alpha$ = 0.2 (dB/cm/MHz). In the case illustrated in FIG. 11, the variance takes a minimum value $S(\alpha)_{min}$ when the attenuation factor candidate value $\alpha$ is 0.2 (dB/cm/MHz). Therefore, in the case illustrated in FIG. 11, the attenuation factor setting unit 334 sets $\alpha$ = 0.2 (dB/cm/MHz) as the optimum attenuation factor.

The feature correction unit 335 then uses the optimum attenuation factor set for each divided region by the attenuation factor setting unit 334 to calculate a cumulative attenuation factor at a sampling point (Step S13). For example, the cumulative attenuation factor $\gamma_{IJ}(h)$ at the sampling point $S_{IJ}(h)$ in the divided region $P_{IJ}$ illustrated in FIG. 6 is expressed by equation (7).

Next, the feature correction unit 335 performs attenuation correction on the features using the cumulative attenuation factor to calculate corrected features (Step S14). For example, the corrected features $a_{IJ}(h)$, $b_{IJ}(h)$, and $c_{IJ}(h)$ of the slope $a_0$, the intercept $b_0$, and the midband fit $c_0$ at the sampling point $S_{IJ}(h)$ in the divided region $P_{IJ}$ illustrated in FIG. 6 are calculated using equations (9) to (11), respectively.

The feature image data generation unit 342 superimposes visual information (for example, hue) associated with the corrected feature calculated in Step S14 on each pixel in the B-mode image data generated by the B-mode image data generation unit 341 to generate feature image data (Step S15). The feature image data generation unit 342 transmits the generated feature image data to the display device 4. The display device 4, which has received the feature image data, displays a feature image corresponding to the received feature image data.

After Step S15, the ultrasound observation apparatus 3 ends a series of the processing steps. The ultrasound observation apparatus 3 repeatedly and periodically executes the processing of Steps S1 to S15.

According to the embodiment of the present invention described above, preliminarily corrected features of a frequency spectrum in accordance with a plurality of attenuation factor candidate values per unit length and per unit frequency that give different attenuation characteristics from each other are calculated in each divided region obtained by dividing an ultrasound image into a plurality of regions. Based on the calculation result, an optimum attenuation factor for an observation target is set for each divided region from the plurality of attenuation factor candidate values. An optimum attenuation factor of a divided region present between the surface of the ultrasound transducer and a sampling point is used to calculate a cumulative attenuation factor per unit frequency at the sampling point. The cumulative attenuation factor is used to perform attenuation correction on features, and thereby corrected features are calculated. Hence, even if the observation target has nonuniform attenuation factors, the corrected features that take the nonuniformity into account can be calculated. Therefore, according to the present invention, it becomes possible to accurately identify the tissue characteristics of the observation target having nonuniform attenuation factors.

Moreover, according to the embodiment, by calculating a sum of an optimum attenuation factor of each divided region present between a surface position of the ultrasound transducer and a sampling point, the optimum attenuation factor being multiplied by a weight indicating a round-trip distance in the depth direction in the divided region, the cumulative attenuation factor at the sampling point is calculated. Accordingly, it is possible to appropriately set an attenuation factor in the middle between the surface of the ultrasound transducer and the sampling point.

Up to this point, the mode for carrying out the present invention has been described. However, the present invention is not intended to be limited only to the above-mentioned embodiment. For example, the method for setting divided regions of an ultrasound image is not limited to the one illustrated in FIG. 6. FIG. 12 is a diagram illustrating another example of the setting of divided regions in an ultrasound image. FIG. 12 illustrates a case where an ultrasound image 201 is divided into 30 divided regions $Q_{ij}$ (i = 1 to 5, j = 1 to 6). Regions at the same depth, that is, regions having a common value in j among the divided regions $Q_{ij}$ all have the same area. On the other hand, regions at different depths among the divided regions $Q_{ij}$ are increased in height $H_j$ (j = 1 to 6) ($H_1 < H_2 < ... < H_6$) as the depth from a surface position 202 of the ultrasound transducer 21 is increased. Generally, ultrasound attenuation is increased with increasing depth. Accordingly, the area of a divided region distant from the ultrasound transducer 21 is increased to enable an improvement in S/N ratio in the distance.

In this case, the cumulative attenuation factor $\gamma_{IJ}(h)$ at the sampling point $S_{IJ}(h)$ whose distance from a boundary on a side close to the ultrasound transducer 21 is h ($0 < h \le H_J$) in the divided region $Q_{IJ}$ ($1 \le I \le i_{max}$, $1 \le J \le j_{max}$; I and j are constants) is expressed as

$$\gamma_{IJ}(h) = [\Sigma_{j=1,2,...,J-1} (2H_j \cdot \alpha(Q_{Ij}))] + 2h \cdot \alpha (Q_{IJ}) \qquad (12).$$

Equation (12) is different from equation (7) in that the length $H_j$ in the direction of depth in the divided region $Q_{Ij}$ of the first term on the right-hand side is dependent on the region.

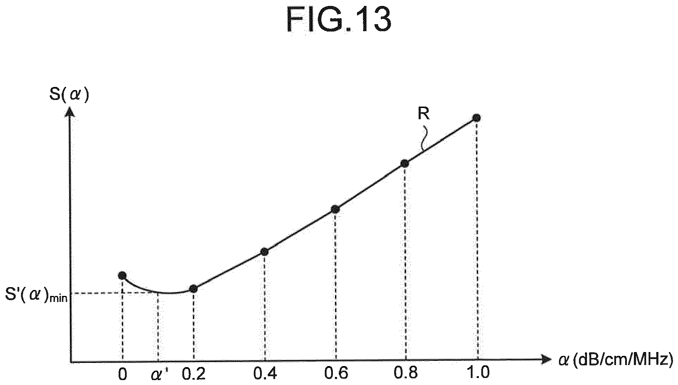

Moreover, the method for the setting of an optimum attenuation factor that is performed by the attenuation factor setting unit 334 is not limited to the above-mentioned method. FIG. 13 is a diagram illustrating an overview of another method for the setting of an optimum attenuation factor that is performed by the attenuation factor setting unit 334. FIG. 13 illustrates an example of the relationship between the attenuation factor candidate value $\alpha$ and the variance $S(\alpha)$, where $\alpha_0 = 0$ (dB/cm/MHz), $\alpha_{max} = 1.0$ (dB/cm/MHz), and $\Delta\alpha = 0.2$ (dB/cm/MHz). All the values of the variance $S(\alpha)$ for the attenuation factor candidate values $\alpha = 0, 0.2, 0.4, 0.6, 0.8$, and $1.0$ (all in dB/cm/MHz) are respectively the same as those in FIG. 11. In this case, the feature calculation unit 333 performs a regression analysis before the attenuation factor setting unit 334 sets an optimum attenuation factor. Accordingly, a curve R that interpolates the values of the variance $S(\alpha)$ for the attenuation factor candidate value $\alpha$ is calculated. The attenuation factor setting unit 334 then calculates a minimum value $S'(\alpha)_{min}$ when $0$ (dB/cm/MHz) $\leq \alpha \leq 1.0$ (dB/cm/MHz) for the curve R and sets a value $\alpha'$ of its attenuation factor candidate value as the optimum attenuation factor. Therefore, in the case illustrated in FIG. 13, the optimum attenuation factor $\alpha'$ is a value between $0$ (dB/cm/MHz) and $0.2$ (dB/cm/MHz).

Moreover, the attenuation factor setting unit 334 may calculate an equivalent optimum attenuation factor value corresponding to an optimum attenuation factor in all frames of the ultrasound image, and set, as the optimum attenuation factor, the mean, median, or mode of a predetermined number of equivalent optimum attenuation factor values including an equivalent optimum attenuation factor value of the latest frame. In this case, the number of changes in optimum attenuation factor is reduced as compared to a case of setting an optimum attenuation factor for each frame, so that the value can be stabilized.

Moreover, the attenuation factor setting unit 334 may set an optimum attenuation factor in predetermined frame intervals of the ultrasound image. Consequently, the amount of computation can be significantly reduced. In this case, it is simply required to use an optimum attenuation factor value that was set last until the next optimum attenuation factor is set.

Moreover, it may be configured such that the input unit 35 can accept the input of a change in the setting of the initial value $\alpha_0$ of the attenuation factor candidate value.

Moreover, it is also possible to apply any of, for example, the standard deviation, the difference between the maximum value and the minimum value of a feature in a population, and the half width of the distribution of features, as the quantity that gives statistical dispersion. A case where the inverse of a variance is applied as the quantity that gives statistical dispersion is also conceivable. In this case, however, it is needless to say that an attenuation factor candidate value having a maximum value is the optimum attenuation factor.

Moreover, it is also possible that the attenuation factor setting unit 334 calculates the statistical dispersion of a plurality of kinds of preliminarily corrected features, and set, as the optimum attenuation factor, an attenuation factor candidate value having minimal statistical dispersion.

Moreover, the attenuation factor setting unit 334 may calculate a preliminarily corrected feature by performing attenuation correction on a frequency spectrum with a plurality of attenuation factor candidate values, and performing a regression analysis on the frequency spectrum after attenuation correction.

Moreover, an application to an ultrasound probe, other than an ultrasound endoscope, is also possible. For example, a slim ultrasound miniature probe without an optical system may be applied as the ultrasound probe. The ultrasound miniature probe is generally used to be inserted into the biliary tract, bile duct, pancreatic duct, trachea, bronchus, urethra, or ureter and observe its surrounding organ (such as the pancreas, lung, prostate, bladder, or lymph node). Moreover, an external ultrasound probe that applies ultrasound from the body surface of a subject may be applied as the ultrasound probe. The external ultrasound probe is generally used to observe an abdominal organ (the liver, gallbladder, or bladder), the breast (especially, the mammary gland), and the thyroid.

In this manner, the present invention can include various embodiments within the range that does not depart from the technical idea described in the claims.

Industrial Applicability

As described above, an ultrasound observation apparatus, a method for operating the ultrasound observation apparatus, and a program for operating the ultrasound observation apparatus according to the present invention are useful for accurately identifying tissue characteristics of an observation target having nonuniform attenuation factors.

Reference Signs List

| | |
|---|---|
| 1 | Ultrasound diagnosis system |
| 2 | Ultrasound endoscope |

| | |
|---|---|
| 3 | Ultrasound observation apparatus |
| 4 | Display device |
| 21 | Ultrasound transducer |
| 31 | Transmitting and receiving unit |
| 32 | Signal processing unit |
| 33 | Computing unit |
| 34 | Image processing unit |
| 35 | Input unit |
| 36 | Control unit |
| 37 | Storage unit |
| 101, 201 | Ultrasound image |
| 102, 202 | Surface position |
| 311 | Signal amplification unit |
| 331 | Amplification correction unit |
| 332 | Frequency analysis unit |
| 333 | Feature calculation unit |
| 334 | Attenuation factor setting unit |
| 335 | Feature correction unit |
| 341 | B-mode image data generation unit |
| 342 | Feature image data generation unit |
| 371 | Divided region information storage unit |
| 372 | Spectrum information storage unit |
| 373 | Feature information storage unit |
| 374 | Attenuation factor information storage unit |

**Claims**

1. An ultrasound observation apparatus (3) for generating an ultrasound image based on an ultrasound signal acquired by an ultrasound probe (2), the ultrasound probe (2) having an ultrasound transducer (21) configured to transmit ultrasound to an observation target and receive the ultrasound reflected from the observation target, the ultrasound observation apparatus (3) comprising:

   a frequency analysis unit (332) configured to analyze a frequency of the ultrasound signal to calculate a plurality of frequency spectra in accordance with reception depths and receiving directions of the ultrasound signal;
   a feature calculation unit (333) configured to calculate features of the plurality of frequency spectra;
   an attenuation factor setting unit (334) configured to use an attenuation factor candidate value per unit length and per unit frequency to perform attenuation correction on the features of the frequency spectra for removing an influence of the ultrasound; and
   a feature correction unit (335) configured to calculate a cumulative attenuation factor per unit frequency at a sampling point, and to perform attenuation correction on the features using the cumulative attenuation factor to calculate a corrected feature,
   **characterized in that**
   the attenuation factor setting unit (334) is configured to use each of a plurality of attenuation factor candidate values per unit length and per unit frequency that give different attenuation characteristics when the ultrasound propagates through the observation target, in each of divided regions obtained by dividing the ultrasound image into a plurality of regions, to perform the attenuation correction on the features of the frequency spectra for removing an influence of the ultrasound, thereby to calculate a preliminarily corrected feature of each of the frequency spectra for each of the attenuation factor candidate values, and to set an optimum attenuation factor for the observation target among the plurality of attenuation factor candidate values based on the calculated preliminarily corrected feature; and
   the feature correction unit (335) is configured to calculate the cumulative attenuation factor per unit frequency at the sampling point, using an optimum attenuation factor of a divided region present between a surface of the ultrasound transducer and the sampling point among optimum attenuation factors set respectively for the divided regions by the attenuation factor setting unit.

2. The ultrasound observation apparatus (3) according to claim 1, wherein the feature correction unit (335) is configured to calculate the cumulative attenuation factor at the sampling point by cumulatively adding the optimum attenuation factors of corresponding ones of the divided regions present between the surface of the ultrasound transducer (21) and the sampling point, the optimum attenuation factor being multiplied by twice the height of the divided regions, and adding to the cumulatively added optimum attenuation factors a product of the optimum attenuation factor of the divided region of the sampling point and a round-trip distance in a depth direction in the divided region of the sampling point.

3. The ultrasound observation apparatus (3) according to claim 1, wherein, in two of the divided regions which are adjacent along a depth direction, one of the two divided regions located more distant from the ultrasound transducer (21) has a length in the depth direction equal to or more than a length in the depth direction of the other of the two divided regions located closer to the ultrasound transducer (21).

4. The ultrasound observation apparatus (3) according to claim 1, wherein the attenuation factor setting unit (334) is configured to calculate a statistical dispersion of the preliminarily corrected feature for each of the attenuation factor candidate values, and set an attenuation factor candidate value having a minimal statistical dispersion as the optimum attenuation factor.

5. The ultrasound observation apparatus (3) according to claim 1, further comprising a feature image data generation unit (342) configured to generate feature image data for showing information on the corrected feature together with the ultrasound image.

6. The ultrasound observation apparatus (3) according to claim 1, wherein the feature calculation unit (333) is configured to approximate each of the frequency spectra by an n-th order polynomial (n is a positive integer) to calculate the features.

7. The ultrasound observation apparatus (3) according to claim 1, wherein
the feature calculation unit (333) is configured to approximate a predetermined frequency band in the frequency spectrum by a linear expression to calculate the features that are one or more of an intercept of the linear expression, a slope of the linear expression, and a midband fit as a value of the linear expression at an intermediate frequency in the frequency band, and that include one of the slope and the midband fit, and
the attenuation factor setting unit (334) is configured to set the optimum attenuation factor based on the one of the slope and the midband fit.

8. The ultrasound observation apparatus (3) according to claim 7, wherein
the attenuation factor setting unit (334) is configured to set the optimum attenuation factor based on the slope when the slope is the features, and set the optimum attenuation factor based on the midband fit when the midband fit is the features.

9. A method for operating an ultrasound observation apparatus (3), the ultrasound observation apparatus (3) being configured to generate an ultrasound image based on an ultrasound signal acquired by an ultrasound probe (2), the ultrasound probe (2) having an ultrasound transducer (21) configured to transmit ultrasound to an observation target and receive the ultrasound reflected from the observation target, the method comprising:

a frequency analysis step of, by a frequency analysis unit (332), analyzing a frequency of the ultrasound signal to calculate a plurality of frequency spectra in accordance with reception depths and receiving directions of the ultrasound signal;
a feature calculation step of, by a feature calculation unit (333), calculating features of the plurality of frequency spectra;
an attenuation factor setting step of, by an attenuation factor setting unit (334), using an attenuation factor candidate value per unit length and per unit frequency to perform attenuation correction on the features of the frequency spectra for removing an influence of the ultrasound; and
a feature correction step of, by a feature correction unit (335), calculating a cumulative attenuation factor per unit frequency at a sampling point, and performing attenuation correction on the features using the cumulative attenuation factor to calculate a corrected feature,
**characterized in that**
the attenuation factor setting step uses each of a plurality of attenuation factor candidate values per unit length and per unit frequency that give different attenuation characteristics when the ultrasound propagates through the observation target, in each of divided regions obtained by dividing the ultrasound image into a plurality of regions, to perform the attenuation correction on the features of the frequency spectra for removing an influence of the ultrasound, thereby calculating a preliminarily corrected feature of each of the frequency spectra for each of the attenuation factor candidate values, and setting an optimum attenuation factor for the observation target among the plurality of attenuation factor candidate values based on the calculated preliminarily corrected feature; and

the feature correction calculates the cumulative attenuation factor per unit frequency at a sampling point by using an optimum attenuation factor of a divided region present between a surface of the ultrasound transducer (21) and the sampling point among optimum attenuation factors set respectively for the divided regions.

10. A program for operating an ultrasound observation apparatus (3), the ultrasound observation apparatus (3) being configured to generate an ultrasound image based on an ultrasound signal acquired by an ultrasound probe (2), the ultrasound probe (2) having an ultrasound transducer (21) configured to transmit ultrasound to an observation target and receive the ultrasound reflected from the observation target, the program causing the ultrasound observation apparatus (3) to execute:

a frequency analysis step of, by a frequency analysis unit (332), analyzing a frequency of the ultrasound signal to calculate a plurality of frequency spectra in accordance with reception depths and receiving directions of the ultrasound signal;
a feature calculation step of, by a feature calculation unit (333), calculating features of the plurality of frequency spectra;
an attenuation factor setting step of, by an attenuation factor setting unit (334), using an attenuation factor candidate value per unit length and per unit frequency to perform attenuation correction on the features of the frequency spectra for removing an influence of the ultrasound; and
a feature correction step of, by a feature correction unit (335), calculating a cumulative attenuation factor per unit frequency at a sampling point, and performing attenuation correction on the features using the cumulative attenuation factor to calculate a corrected feature,
**characterized in that**
the attenuation factor setting step uses each of a plurality of attenuation factor candidate values per unit length and per unit frequency that give different attenuation characteristics when the ultrasound propagates through the observation target, in each of divided regions obtained by dividing the ultrasound image into a plurality of regions, to perform the attenuation correction on the features of the frequency spectra for removing an influence of the ultrasound, thereby calculating a preliminarily corrected feature of each of the frequency spectra for each of the attenuation factor candidate values, and setting an optimum attenuation factor for the observation target among the plurality of attenuation factor candidate values based on the calculated preliminarily corrected feature; and
the feature correction step calculates the cumulative attenuation factor per unit frequency at a sampling point by using an optimum attenuation factor of a divided region present between a surface of the ultrasound transducer (21) and the sampling point among optimum attenuation factors set respectively for the divided regions.

**Patentansprüche**

1. Ultraschallbeobachtungsgerät (3) zum Erzeugen eines Ultraschallbildes basierend auf einem Ultraschallsignal, das durch eine Ultraschallsonde (2) erfasst wird, wobei die Ultraschallsonde (2) einen Ultraschallwandler (21) aufweist, der konfiguriert ist, um Ultraschall an ein Beobachtungsziel zu senden und den Ultraschall, der von dem Beobachtungsziel reflektiert wird, zu empfangen, wobei das Ultraschallbeobachtungsgerät (3) umfasst:

eine Frequenzanalyseeinheit (332), die konfiguriert ist, um eine Frequenz des Ultraschallsignals zu analysieren, um eine Mehrzahl von Frequenzspektren gemäß den Empfangstiefen und den Empfangsrichtungen des Ultraschallsignals zu berechnen;
eine Merkmalsberechnungseinheit (333), die konfiguriert ist, um Merkmale der Mehrzahl von Frequenzspektren zu berechnen;
eine Einheit (334) zum Einstellen eines Dämpfungsfaktors, die konfiguriert ist, um einen Kandidatenwert des Dämpfungsfaktors pro Längeneinheit und pro Frequenzeinheit zu verwenden, um eine Dämpfungskorrektur an den Merkmalen der Frequenzspektren auszuführen, um einen Einfluss des Ultraschalls zu beseitigen; und
eine Merkmalskorrektureinheit (335), die konfiguriert ist, um einen kumulativen Dämpfungsfaktor pro Frequenzeinheit an einem Abtastpunkt zu berechnen und um eine Dämpfungskorrektur an den Merkmalen unter Verwendung des kumulativen Dämpfungsfaktors auszuführen, um ein korrigiertes Merkmal zu berechnen,
**dadurch gekennzeichnet, dass**

die Einheit (334) zum Einstellen eines Dämpfungsfaktors konfiguriert ist, um jeden von einer Mehrzahl von Kandidatenwerten des Dämpfungsfaktors pro Längeneinheit und pro Frequenzeinheit, die unterschiedliche Dämpfungskennzeichen ergeben, wenn sich der Ultraschall durch das Beobachtungsziel hindurch ausbreitet, in jeder der unterteilten Regionen, die durch Unterteilen des Ultraschallbildes in eine Mehrzahl von Regionen erzielt werden, zu verwenden, um die Dämpfungskorrektur an den Merkmalen der Frequenzspektren auszuführen, um einen Einfluss des Ultraschalls zu beseitigen, um dadurch ein vorläufig korrigiertes Merkmal jedes der Frequenzspektren für jeden der Kandidatenwerte des Dämpfungsfaktors zu berechnen, und um einen optimalen Dämpfungsfaktor für das Beobachtungsziel aus der Mehrzahl von Kandidatenwerten des Dämpfungsfaktors basierend auf dem berechneten vorläufig korrigierten Merkmal einzustellen; und

die Merkmalskorrektureinheit (335) konfiguriert ist, um den kumulativen Dämpfungsfaktor pro Frequenzeinheit an dem Abtastpunkt zu berechnen, indem sie einen optimalen Dämpfungsfaktor einer unterteilten Region, die zwischen einer Oberfläche des Ultraschallwandlers und dem Abtastung vorhanden ist, aus optimalen Dämpfungsfaktoren, die jeweils für die unterteilten Regionen durch die Einheit zum Einstellen eines Dämpfungsfaktors eingestellt werden, verwendet.

2. Ultraschallbeobachtungsgerät (3) nach Anspruch 1, wobei die Merkmalskorrektureinheit (335) konfiguriert ist, um den kumulativen Dämpfungsfaktor an dem Abtastpunkt zu berechnen, indem sie die optimalen Dämpfungsfaktoren von entsprechenden der unterteilten Regionen, die zwischen der Oberfläche des Ultraschallwandlers (21) und dem Abtastpunkt vorhanden sind, kumulativ addiert, wobei der optimale Dämpfungsfaktor mit der doppelten Höhe der unterteilten Regionen multipliziert wird, und indem sie zu den kumulativ addierten optimalen Dämpfungsfaktoren ein Produkt des optimalen Dämpfungsfaktors der unterteilten Region des Abtastpunkts und einer Umlaufentfernung in einer Tiefenrichtung in der unterteilten Region des Abtastpunktes addiert.

3. Ultraschallbeobachtungsgerät (3) nach Anspruch 1, wobei zweien der unterteilten Regionen, die entlang einer Tiefenrichtung nebeneinanderliegen eine der beiden unterteilten Regionen, die sich weiter von dem Ultraschallwandler (21) entfernt befindet, eine Länge in der Tiefenrichtung aufweist, die gleich oder größer als eine Länge in der Tiefenrichtung der anderen der beiden unterteilten Regionen, die sich näher an dem Ultraschallwandler (21) befindet, ist.

4. Ultraschallbeobachtungsgerät (3) nach Anspruch 1, wobei die Einheit (334) zum Einstellen eines Dämpfungsfaktors konfiguriert ist, um eine statistische Streuung des vorläufig korrigierten Merkmals für jeden der Kandidatenwerte des Dämpfungsfaktors zu berechnen, und um einen Kandidatenwert des Dämpfungsfaktors, der eine minimale statistische Streuung aufweist, als den optimalen Dämpfungsfaktor einzustellen.

5. Ultraschallbeobachtungsgerät (3) nach Anspruch 1, ferner umfassend eine Einheit (342) zum Erzeugen von Merkmalsbilddaten, die konfiguriert ist, um Merkmalsbilddaten zu erzeugen, um Informationen über das korrigierte Merkmal zusammen mit dem Ultraschallbild zu erzeugen.

6. Ultraschallbeobachtungsgerät (3) nach Anspruch 1, wobei die Merkmalsberechnungseinheit (333) konfiguriert ist, um jedes der Frequenzspektren mit einem Polynom n. Ordnung (n ist eine positive Ganzzahl) zu nähern, um die Merkmale zu berechnen.

7. Ultraschallbeobachtungsgerät (3) nach Anspruch 1, wobei
die Merkmalsberechnungseinheit (333) konfiguriert ist, um ein vorbestimmtes Frequenzband in dem Frequenzspektrum durch einen linearen Ausdruck zu nähern, um die Merkmale zu berechnen, die eines oder mehrere sind von einem Achsabschnitt des linearen Ausdrucks, einem Richtungskoeffizienten des linearen Ausdrucks und einer Bandmittenanpassung als Wert des linearen Ausdrucks auf einer Zwischenfrequenz in dem Frequenzband, und die eines von dem Richtungskoeffizienten und der Bandmittenanpassung umfassen, und
die Einheit (334) zum Einstellen eines Dämpfungsfaktors konfiguriert ist, um den optimalen Dämpfungsfaktor basierend auf dem einen von dem Richtungskoeffizienten und der Bandmittenanpassung einzustellen.

8. Ultraschallbeobachtungsgerät (3) nach Anspruch 7, wobei die Einheit (334) zum Einstellen eines Dämpfungsfaktors konfiguriert ist, um den optimalen Dämpfungsfaktor basierend auf dem Richtungskoeffizienten einzustellen, wenn der Richtungskoeffizient das Merkmal ist, und um den optimalen Dämpfungsfaktor basierend auf der Bandmittenanpassung einzustellen, wenn die Bandmittenanpassung das Merkmal ist.

9. Verfahren zum Betätigen eines Ultraschallbeobachtungsgeräts (3), wobei das Ultraschallbeobachtungsgerät (3) konfiguriert ist, um ein Ultraschallbild basierend auf einem Ultraschallsignal, das durch eine Ultraschallsonde (2) erfasst wird, zu erzeugen, wobei die Ultraschallsonde (2) einen Ultraschallwandler (21) aufweist, der konfiguriert ist, um Ultraschall an ein Beobachtungsziel zu senden und den Ultraschall, der von dem Beobachtungsziel reflektiert wird, zu empfangen, wobei das Verfahren umfasst:

einen Frequenzanalyseschritt, der darin besteht, durch eine Frequenzanalyseeinheit (332) eine Frequenz des Ultraschallsignals zu analysieren, um eine Mehrzahl von Frequenzspektren gemäß den Empfangstiefen und den Empfangsrichtungen des Ultraschallsignals zu berechnen;

einen Merkmalsberechnungsschritt, der darin besteht, durch eine Merkmalsberechnungseinheit (333) Merkmale der Mehrzahl von Frequenzspektren zu berechnen;

einen Schritt zum Einstellen eines Dämpfungsfaktors, der darin besteht, durch eine Einheit (334) zum Einstellen eines Dämpfungsfaktors einen Kandidatenwert des Dämpfungsfaktors pro Längeneinheit und pro Frequenzeinheit zu verwenden, um eine Dämpfungskorrektur an den Merkmalen der Frequenzspektren auszuführen, um einen Einfluss des Ultraschalls zu beseitigen; und

einen Merkmalskorrekturschritt, der darin besteht, durch eine Merkmalskorrektureinheit (335) einen kumulativen Dämpfungsfaktor pro Frequenzeinheit an einem Abtastpunkt zu berechnen, und eine Dämpfungskorrektur an den Merkmalen unter Verwendung des kumulativen Dämpfungsfaktors auszuführen, um ein korrigiertes Merkmal zu berechnen,

**dadurch gekennzeichnet, dass**

der Schritt zum Einstellen eines Dämpfungsfaktors jeden von einer Mehrzahl von Kandidatenwerten des Dämpfungsfaktors pro Längeneinheit und pro Frequenzeinheit, die unterschiedliche Dämpfungskennzeichen ergeben, wenn sich der Ultraschall durch das Beobachtungsziel hindurch ausbreitet, in jeder der unterteilten Regionen, die durch Unterteilen des Ultraschallbildes in eine Mehrzahl von Regionen erzielt werden, zu verwenden, um die Dämpfungskorrektur an den Merkmalen der Frequenzspektren auszuführen, um einen Einfluss des Ultraschalls zu beseitigen, um dadurch ein vorläufig korrigiertes Merkmal jedes der Frequenzspektren für jeden der Kandidatenwerte des Dämpfungsfaktors zu berechnen, und um einen optimalen Dämpfungsfaktor für das Beobachtungsziel aus der Mehrzahl von Kandidatenwerten des Dämpfungsfaktors basierend auf dem berechneten vorläufig korrigierten Merkmal einzustellen; und

die Merkmalskorrektur den kumulativen Dämpfungsfaktor pro Frequenzeinheit an einem Abtastpunkt berechnet, indem ein optimaler Dämpfungsfaktor einer unterteilten Region, die zwischen einer Oberfläche des Ultraschallwandlers (21) und dem Abtastpunkt vorhanden ist, aus optimalen Dämpfungsfaktoren, die jeweils für die unterteilten Regionen eingestellt werden, verwendet wird.

10. Programm zum Betätigen eines Ultraschallbeobachtungsgeräts (3), wobei das Ultraschallbeobachtungsgerät (3) konfiguriert ist, um ein Ultraschallbild basierend auf einem Ultraschallsignal, das durch eine Ultraschallsonde (2) erfasst wird, zu erzeugen, wobei die Ultraschallsonde (2) einen Ultraschallwandler (21) aufweist, der konfiguriert ist, um Ultraschall an ein Beobachtungsziel zu senden und den Ultraschall, der von dem Beobachtungsziel reflektiert wird, zu empfangen, wobei das Programm bewirkt, dass das Ultraschallbeobachtungsgerät (3) ausführt:

einen Frequenzanalyseschritt, der darin besteht, durch eine Frequenzanalyseeinheit (332) eine Frequenz des Ultraschallsignals zu analysieren, um eine Mehrzahl von Frequenzspektren gemäß den Empfangstiefen und den Empfangsrichtungen des Ultraschallsignals zu berechnen;

einen Merkmalsberechnungsschritt, der darin besteht, durch eine Merkmalsberechnungseinheit (333) Merkmale der Mehrzahl von Frequenzspektren zu berechnen;

einen Schritt zum Einstellen eines Dämpfungsfaktors, der darin besteht, durch eine Einheit (334) zum Einstellen eines Dämpfungsfaktors einen Kandidatenwert des Dämpfungsfaktors pro Längeneinheit und pro Frequenzeinheit zu verwenden, um eine Dämpfungskorrektur an den Merkmalen der Frequenzspektren auszuführen, um einen Einfluss des Ultraschalls zu beseitigen; und

einen Merkmalskorrekturschritt, der darin besteht, durch eine Merkmalskorrektureinheit (335) einen kumulativen Dämpfungsfaktor pro Frequenzeinheit an einem Abtastpunkt zu berechnen, und eine Dämpfungskorrektur an den Merkmalen unter Verwendung des kumulativen Dämpfungsfaktors auszuführen, um ein korrigiertes Merkmal zu berechnen,

**dadurch gekennzeichnet, dass**

der Schritt zum Einstellen eines Dämpfungsfaktors jeden von einer Mehrzahl von Kandidatenwerten des Dämpfungsfaktors pro Längeneinheit und pro Frequenzeinheit, die unterschiedliche Dämpfungskennzeichen ergeben, wenn sich der Ultraschall durch das Beobachtungsziel hindurch ausbreitet, in jeder der unterteilten Regionen, die durch Unterteilen des Ultraschallbildes in eine Mehrzahl von Regionen erzielt werden, zu verwenden, um die Dämpfungskorrektur an den Merkmalen der Frequenzspektren auszuführen, um einen Einfluss des Ultraschalls zu beseitigen, um dadurch ein vorläufig korrigiertes Merkmal jedes der Frequenzspektren für jeden der Kandidatenwerte des Dämpfungsfaktors zu berechnen, und um einen optimalen Dämpfungsfaktor für das Beobachtungsziel aus der Mehrzahl von Kandidatenwerten des Dämpfungsfaktors basierend auf dem berechneten vorläufig korrigierten Merkmal einzustellen; und

der Merkmalskorrekturschritt den kumulativen Dämpfungsfaktor pro Frequenzeinheit an einem Abtastpunkt berechnet, indem ein optimaler Dämpfungsfaktor einer unterteilten Region, die zwischen einer Oberfläche des Ultraschallwandlers (21) und dem Abtastpunkt vorhanden ist, aus optimalen Dämpfungsfaktoren, die jeweils für die unterteilten Regionen eingestellt werden, verwendet wird.

## Revendications

1. Appareil d'observation ultrasonore (3) pour générer une image ultrasonore sur la base d'un signal ultrasonore acquis par une sonde ultrasonore (2), la sonde ultrasonore (2) ayant un transducteur ultrasonore (21) configuré pour émettre un ultrason vers une cible d'observation et recevoir l'ultrason réfléchi par la cible d'observation, l'appareil d'observation ultrasonore (3) comprenant :

une unité d'analyse de fréquence (332) configurée pour analyser une fréquence du signal ultrasonore pour calculer une pluralité de spectres de fréquence selon des profondeurs de réception et des directions de réception du signal ultrasonore ;

une unité de calcul de caractéristiques (333) configurée pour calculer des caractéristiques de la pluralité de spectres de fréquence ;

une unité de réglage de facteur d'atténuation (334) configurée pour utiliser une valeur candidate de facteur d'atténuation par longueur unitaire et par fréquence unitaire pour réaliser une correction d'atténuation sur les caractéristiques des spectres de fréquence afin d'éliminer une influence de l'ultrason ; et

une unité de correction de caractéristiques (335) configurée pour calculer un facteur d'atténuation cumulatif par fréquence unitaire au niveau d'un point d'échantillonnage, et pour réaliser une correction d'atténuation sur les caractéristiques à l'aide du facteur d'atténuation cumulatif pour calculer une caractéristique corrigée,

**caractérisé par le fait que**

l'unité de réglage de facteur d'atténuation (334) est configurée pour utiliser chacune d'une pluralité de valeurs candidates de facteur d'atténuation par longueur unitaire et par fréquence unitaire qui donnent des caractéristiques d'atténuation différentes lorsque l'ultrason se propage à travers la cible d'observation, dans chacune de régions divisées obtenues par division de l'image ultrasonore en une pluralité de régions, pour réaliser la correction d'atténuation sur les caractéristiques des spectres de fréquence afin d'éliminer une influence de l'ultrason, ainsi pour calculer une caractéristique corrigée de façon préliminaire de chacun des spectres de fréquence pour chacune des valeurs candidates de facteur d'atténuation, et pour régler un facteur d'atténuation optimal pour la cible d'observation parmi la pluralité de valeurs candidates de facteur d'atténuation sur la base de la caractéristique corrigée de façon préliminaire calculée ; et

l'unité de correction de caractéristiques (335) est configurée pour calculer le facteur d'atténuation cumulatif par fréquence unitaire au niveau du point d'échantillonnage, à l'aide d'un facteur d'atténuation optimal d'une région divisée présente entre une surface du transducteur ultrasonore et le point d'échantillonnage parmi des facteurs d'atténuation optimaux réglés respectivement pour les régions divisées par l'unité de réglage de facteur d'atténuation.

2. Appareil d'observation ultrasonore (3) selon la revendication 1, dans lequel l'unité de correction de caractéristiques (335) est configurée pour calculer le facteur d'atténuation cumulatif au niveau du point d'échantillonnage par ajout de façon cumulative des facteurs d'atténuation optimaux de régions divisées correspondantes parmi les régions divisées présentes entre la surface du transducteur ultrasonore (21) et le point d'échantillonnage, le facteur d'atténuation optimal étant multiplié par deux fois la hauteur des régions divisées, et ajout aux facteurs d'atténuation optimaux ajoutés de façon cumulative d'un produit du facteur d'atténuation optimal de la région divisée du point d'échantillonnage et d'une distance aller-retour dans une direction de profondeur dans la région divisée du point d'échantillonnage.

3. Appareil d'observation ultrasonore (3) selon la revendication 1, dans lequel, dans deux des régions divisées qui sont adjacentes le long d'une direction de profondeur, l'une des deux régions divisées situées de manière plus distante du transducteur ultrasonore (21) possède une longueur dans la direction de profondeur égale ou supérieure à une longueur dans la direction de profondeur de l'autre des deux régions divisées située de manière plus proche du transducteur ultrasonore (21).

4. Appareil d'observation ultrasonore (3) selon la revendication 1, dans lequel l'unité de réglage de facteur d'atténuation (334) est configurée pour calculer une dispersion statistique de la caractéristique corrigée de façon préliminaire pour chacune des valeurs candidates de facteur d'atténuation, et régler une valeur candidate de facteur d'atténuation ayant une dispersion statistique minimale en tant que facteur d'atténuation optimal.

5. Appareil d'observation ultrasonore (3) selon la revendication 1, comprenant en outre une unité de génération de données d'image caractéristique (342) configurée pour générer des données d'image caractéristique pour représenter des informations sur la caractéristique corrigée conjointement avec l'image ultrasonore.

6. Appareil d'observation ultrasonore (3) selon la revendication 1, dans lequel l'unité de calcul de caractéristiques (333) est configurée pour réaliser une approximation de chacun des spectres de fréquence par un polynôme d'ordre n (n est un nombre entier positif) pour calculer les caractéristiques.

7. Appareil d'observation ultrasonore (3) selon la revendication 1, dans lequel l'unité de calcul de caractéristiques (333) est configurée pour réaliser une approximation d'une bande de fréquences prédéterminée dans le spectre de fréquence par une expression linéaire pour calculer les caractéristiques qui sont un ou plusieurs parmi un point intersection de l'expression linéaire, une pente de l'expression linéaire, et un ajustement de bande intermédiaire en tant que valeur de l'expression linéaire à une fréquence intermédiaire dans la bande de fréquences, et qui comprennent l'un parmi la pente et l'ajustement de bande intermédiaire, et
l'unité de réglage de facteur d'atténuation (334) est configurée pour régler le facteur d'atténuation optimal sur la base de l'un parmi la pente et de l'ajustement de bande intermédiaire.

8. Appareil d'observation ultrasonore (3) selon la revendication 7, dans lequel
l'unité de réglage de facteur d'atténuation (334) est configurée pour régler le facteur d'atténuation optimal sur la base de la pente lorsque la pente constitue les caractéristiques, et régler le facteur d'atténuation optimal sur la base de l'ajustement de bande intermédiaire lorsque l'ajustement de bande intermédiaire constitue les caractéristiques.

9. Procédé de fonctionnement d'un appareil d'observation ultrasonore (3), l'appareil d'observation ultrasonore (3) étant configuré pour générer une image ultrasonore sur la base d'un signal ultrasonore acquis par une sonde ultrasonore (2), la sonde ultrasonore (2) présentant un transducteur ultrasonore (21) configuré pour émettre un ultrason vers une cible d'observation et recevoir l'ultrason réfléchi par la cible d'observation, le procédé comprenant :

une étape d'analyse de fréquence consistant à, par une unité d'analyse de fréquence (332), analyser une fréquence du signal ultrasonore pour calculer une pluralité de spectres de fréquence selon des profondeurs de réception et des directions de réception du signal ultrasonore ;
une étape de calcul de caractéristiques consistant à, par une unité de calcul de caractéristiques (333), calculer des caractéristiques de la pluralité de spectres de fréquence ;
une étape de réglage de facteur d'atténuation consistant à, par une unité de réglage de facteur d'atténuation (334), utiliser une valeur candidate de facteur d'atténuation par longueur unitaire et par fréquence unitaire pour réaliser une correction d'atténuation sur les caractéristiques des spectres de fréquence afin d'éliminer une influence de l'ultrason ; et
une étape de correction de caractéristiques consistant à, par une unité de correction de caractéristiques (335), calculer un facteur d'atténuation cumulatif par fréquence unitaire au niveau d'un point d'échantillonnage, et réaliser une correction d'atténuation sur les caractéristiques à l'aide du facteur d'atténuation cumulatif pour calculer une caractéristique corrigée,
**caractérisé par le fait que**
l'étape de réglage de facteur d'atténuation utilise chacune d'une pluralité de valeurs candidates de facteur d'atténuation par longueur unitaire et par fréquence unitaire qui donnent des caractéristiques d'atténuation différentes lorsque l'ultrason se propage à travers la cible d'observation, dans chacune de régions divisées obtenues par division de l'image ultrasonore en une pluralité de régions, pour réaliser la correction d'atténuation sur les caractéristiques des spectres de fréquence afin d'éliminer une influence de l'ultrason, calculant ainsi une caractéristique corrigée de façon préliminaire de chacun des spectres de fréquence pour chacune des valeurs candidates de facteur d'atténuation, et réglant un facteur d'atténuation optimal pour la cible d'observation parmi la pluralité de valeurs candidates de facteur d'atténuation sur la base de la caractéristique corrigée de façon préliminaire calculée ; et
la correction de caractéristiques calcule le facteur d'atténuation cumulatif par fréquence unitaire au niveau d'un point d'échantillonnage à l'aide d'un facteur d'atténuation optimal d'une région divisée présente entre une surface du transducteur ultrasonore (21) et le point d'échantillonnage parmi des facteurs d'atténuation optimaux réglés respectivement pour les régions divisées.

10. Programme de fonctionnement d'un appareil d'observation ultrasonore (3), l'appareil d'observation ultrasonore (3) étant configuré pour générer une image ultrasonore sur la base d'un signal ultrasonore acquis par une sonde ultrasonore (2), la sonde ultrasonore (2) présentant un transducteur ultrasonore (21) configuré pour émettre un ultrason vers une cible d'observation et recevoir l'ultrason réfléchi par la cible d'observation, le programme amenant l'appareil d'observation ultrasonore (3) à exécuter :

une étape d'analyse de fréquence consistant à, par une unité d'analyse de fréquence (332), analyser une fréquence du signal ultrasonore pour calculer une pluralité de spectres de fréquence selon des profondeurs de réception et des directions de réception du signal ultrasonore ;
une étape de calcul de caractéristiques consistant à, par une unité de calcul de caractéristiques (333), calculer des caractéristiques de la pluralité de spectres de fréquence ;
une étape de réglage de facteur d'atténuation consistant à, par une unité de réglage de facteur d'atténuation (334), utiliser une valeur candidate de facteur d'atténuation par longueur unitaire et par fréquence unitaire pour réaliser une correction d'atténuation sur les caractéristiques des spectres de fréquence afin d'éliminer une influence de l'ultrason ; et
une étape de correction de caractéristiques consistant à, par une unité de correction de caractéristiques (335), calculer un facteur d'atténuation cumulatif par fréquence unitaire au niveau d'un point d'échantillonnage, et réaliser une correction d'atténuation sur les caractéristiques à l'aide du facteur d'atténuation cumulatif pour calculer une caractéristique corrigée,
**caractérisé par le fait que**
l'étape de réglage de facteur d'atténuation utilise chacune d'une pluralité de valeurs candidates de facteur d'atténuation par longueur unitaire et par fréquence unitaire qui donnent des caractéristiques d'atténuation différentes lorsque l'ultrason se propage à travers la cible d'observation, dans chacune de régions divisées obtenues par division de l'image ultrasonore en une pluralité de régions, pour réaliser la correction d'atténuation sur les caractéristiques des spectres de fréquence afin d'éliminer une influence de l'ultrason, calculant ainsi une caractéristique corrigée de façon préliminaire de chacun des spectres de fréquence pour chacune des valeurs candidates de facteur d'atténuation, et réglant un facteur d'atténuation optimal pour la cible d'observation parmi la pluralité de valeurs candidates de facteur d'atténuation sur la base de la caractéristique corrigée de façon préliminaire calculée ; et
l'étape de correction de caractéristiques calcule le facteur d'atténuation cumulatif par fréquence unitaire au niveau d'un point d'échantillonnage à l'aide d'un facteur d'atténuation optimal d'une région divisée présente entre une surface du transducteur ultrasonore (21) et le point d'échantillonnage parmi des facteurs d'atténuation optimaux réglés respectivement pour les régions divisées.

# FIG.1

EP 3 238 632 B1

# FIG.2

# FIG.3

# FIG.4

SHALLOW $\longrightarrow$ DEEP

$Z^{(k)}_0$    D    $SR_k$    $Z^{(k)}_{max}$

$F_1$

$F_2$

$F_3$

$F_{K-1}$

$F_K$

# FIG.5

# FIG.6

# FIG.7

# FIG.8

# FIG.9

```
                    ┌──────────────┐
                    │    START     │
                    └──────┬───────┘
                           ▼
┌──────────────────────────────────────────────────────┐
│  RECEIVE MEASUREMENT RESULT OF OBSERVATION TARGET      │──S1
└──────────────────────────┬───────────────────────────┘
                           ▼
┌──────────────────────────────────────────────────────┐
│     AMPLIFY RECEIVED ECHO SIGNAL (STC CORRECTION)      │──S2
└──────────────────────────┬───────────────────────────┘
                           ▼
┌──────────────────────────────────────────────────────┐
│            GENERATE B-MODE IMAGE DATA                  │──S3
└──────────────────────────┬───────────────────────────┘
                           ▼
┌──────────────────────────────────────────────────────┐
│             CORRECT AMPLIFICATION                      │──S4
└──────────────────────────┬───────────────────────────┘
                           ▼
┌──────────────────────────────────────────────────────┐
│             FREQUENCY ANALYSIS                         │──S5
└──────────────────────────┬───────────────────────────┘
                           ▼
┌──────────────────────────────────────────────────────┐
│              CALCULATE FEATURES                        │──S6
└──────────────────────────┬───────────────────────────┘
                           ▼
┌──────────────────────────────────────────────────────┐
│                 $\alpha = \alpha_0$                    │──S7
└──────────────────────────┬───────────────────────────┘
                           ▼
┌──────────────────────────────────────────────────────┐
│  PERFORM ATTENUATION CORRECTION ON FEATURE            │──S8
│  USING α AS ATTENUATION FACTOR CANDIDATE VALUE        │
└──────────────────────────┬───────────────────────────┘
                           ▼
┌──────────────────────────────────────────────────────┐
│  CALCULATE VARIANCE IN PRELIMINARILY CORRECTED FEATURE│──S9
│         AFTER ATTENUATION CORRECTION                   │
└──────────────────────────┬───────────────────────────┘
                           ▼
┌──────────────────────────────────────────────────────┐
│              $\alpha = \alpha + \Delta\alpha$          │──S10
└──────────────────────────┬───────────────────────────┘
                           ▼
                    ╱─────────────╲  S11
                   ╱ $\alpha > \alpha_{max}$? ╲───── NO
                    ╲─────────────╱
                          │ YES
                          ▼
┌──────────────────────────────────────────────────────┐
│ SET, AS OPTIMUM ATTENUATION FACTOR, ATTENUATION FACTOR│──S12
│ CANDIDATE VALUE HAVING MINIMUM VARIANCE IN PRELIMINARILY│
│      CORRECTED FEATURE IN EACH DIVIDED REGION          │
└──────────────────────────┬───────────────────────────┘
                           ▼
┌──────────────────────────────────────────────────────┐
│  CALCULATE CUMULATIVE ATTENUATION FACTOR AT EACH      │──S13
│              SAMPLING POINT                            │
└──────────────────────────┬───────────────────────────┘
                           ▼
┌──────────────────────────────────────────────────────┐
│  PERFORM ATTENUATION CORRECTION ON FEATURE USING      │──S14
│         CUMULATIVE ATTENUATION FACTOR                  │
└──────────────────────────┬───────────────────────────┘
                           ▼
┌──────────────────────────────────────────────────────┐
│           GENERATE FEATURE IMAGE DATA                  │──S15
└──────────────────────────┬───────────────────────────┘
                           ▼
                    ┌──────────────┐
                    │     END      │
                    └──────────────┘
```

# FIG.10

```
        ┌─────────────────────┐
        │      FREQUENCY       │
        │      ANALYSIS        │
        └─────────────────────┘
                   │
        ┌─────────────────────┐
        │        k=k₀          │  ~S21
        └─────────────────────┘
```

$k=k_0$ — S21

$Z^{(k)}=Z^{(k)}_0$ — S22

ACQUIRE SAMPLE DATA GROUP — S23

APPLY WINDOW FUNCTION — S24

IS SAMPLE DATA GROUP NORMAL? — S25

NO

INSERT ZERO DATA TO COVER SHORTFALL — S26

YES

FFT COMPUTATION — S27

$Z^{(k)}=Z^{(k)}+D$ — S28

$Z^{(k)}>Z^{(k)}_{max}$? — S29

NO

YES

$k=k+1$ — S30

$k>k_{max}$? — S31

NO

YES

RETURN

# FIG.11

# FIG.12

# FIG.13

**EP 3 238 632 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2013035594 A1
- JP 2010246640 A